(19) Europäisches Patentamt
European Patent Office
Office européen des brevets

(11) EP 2 536 725 B1

(12) **EUROPEAN PATENT SPECIFICATION**

(45) Date of publication and mention of the grant of the patent:
**28.10.2015 Bulletin 2015/44**

(21) Application number: **11705850.3**

(22) Date of filing: **16.02.2011**

(51) Int Cl.:
***C07D 475/04*** (2006.01) ***A01N 43/54*** (2006.01)
***A61P 29/00*** (2006.01) ***A61P 35/00*** (2006.01)

(86) International application number:
**PCT/EP2011/052280**

(87) International publication number:
**WO 2011/101369 (25.08.2011 Gazette 2011/34)**

(54) **DIHYDROPTERIDINONES, METHOD FOR PRODUCTION AND USE THEREOF**

DIHYDROPTERIDINONE, HERSTELLUNGSVERFAHREN DAFÜR UND VERWENDUNG DAVON

DIHYDROPTÉRIDINONES, PROCÉDÉ POUR LA PRODUCTION ET L'UTILISATION DE CELLES-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.02.2010 EP 10153827**

(43) Date of publication of application:
**26.12.2012 Bulletin 2012/52**

(73) Proprietor: **Boehringer Ingelheim International GmbH**
**55216 Ingelheim am Rhein (DE)**

(72) Inventors:
- **LINZ, Guenter**
  **55216 Ingelheim am Rhein (DE)**
- **BISCHOFF, Daniel**
  **55216 Ingelheim am Rhein (DE)**
- **EBNER, Thomas**
  **55216 Ingelheim am Rhein (DE)**

(74) Representative: **Dieminger, Wilhelm Gotthard et al**
**Boehringer Ingelheim GmbH**
**Corporate Patents**
**Binger Strasse 173**
**55216 Ingelheim am Rhein (DE)**

(56) References cited:
**WO-A1-03/020722** **WO-A1-2004/076454**
**WO-A1-2006/005510** **WO-A1-2006/018182**
**WO-A1-2006/021378** **WO-A1-2006/021379**
**WO-A1-2006/021547** **WO-A1-2009/112524**
**WO-A2-2006/018185**

- **D. Rudolph ET AL: "BI 6727, A Polo-like Kinase Inhibitor with Improved Pharmacokinetic Profile and Broad Antitumor Activity", Clinical Cancer Research, vol. 15, no. 9, 1 May 2009 (2009-05-01), pages 3094-3102, XP055121606, ISSN: 1078-0432, DOI: 10.1158/1078-0432.CCR-08-2445**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to new dihydropteridinones of general formula (12)

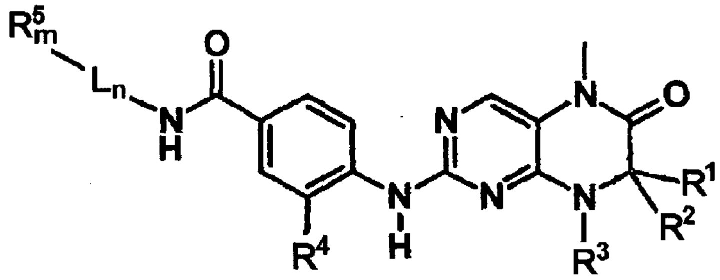

wherein the groups $R^1$, $R^2$, $R^3$, $R^4$, $R^5$, and L, n and m have the meanings given in the claims and specification, optionally in form of its hydrates, tautomers, racemates, enantiomers, diastereomers and the mixtures thereof and optionally the salts thereof, a method of production and the use thereof, particularly in cancer therapy.

### Background to the invention

Polo-like kinases (PLKs) are serine/ threonine kinases that play important roles in regulating processes in the cell cycle. There are 4 PLKs disclosed in the state of the art, i.e. PLK-1, PLK-2, PLK-3 and PLK-4. PLKs play a role in the regulation of the eukaryotic cell cycle (e.g. regulation of the mitotic machinery in mammalian cells). Especially for PLK-1 a central role with respect to the regulation of mitosis is shown (Glover et al. 1998, Genes Dev. 12: 3777-87.; Qian et al. 2001, Mol Biol Cell. 12: 1791-9). Overexpression of PLK-1 seems to be strongly associated with neoplastic cells including cancer (WO 2004/014899). Overexpression of PLK-1 has been documented for various tumour types such as non-small cell lung cancer, squamous cell carcinomas, breast, ovary or papillary carcinomas as well as colorectal cancers (Wolf et al. 1997, Oncogene 14, pp. 543-549; Knecht et al. 1999, Cancer Res. 59, pages 2794-2797; Wolf et al. 2000, Pathol Res Pract. 196, pp. 753-759; Weichert et al. 2004, Br. J. Cancer 90, pp. 815-821; Ito et al. 2004, Br. J. Cancer 90, pp. 414-418; Takahashi et al. 2003, Cancer Sci. 94, pp. 148-152).

Furthermore, pteridinone derivatives are known from the prior art, for example, from the following documents:

WO 01/019825 A1, WO 06/021547, WO 06/021378, WO 06/021379, WO 06/005510 and WO 09/112524 describes specific pteridinones having a different structure than those of the present invention. These compounds are described to be potent inhibitors of cyclin-dependent kinases (cdks) and growth-factor-mediated kinases and are used for the treatment of cell proliferative diseases and disorders, particularly tumour and viral diseases.

WO 2004/076454 A1 and WO 03/020722 A1 disclose specific pteridinones, methods for the production and use thereof. The compounds are descibed to posess antiproliferative activity and are used for preparing a pharmaceutical composition for the treatment and/or prevention of cancer, infections, inflammatory and autoimmune diseases. The present invention is a selection invention over WO 03/020722 A1, the substituents of $R^9$ defined in WO 03/020722 A1 may be selected from among OH, $OCH_3$, Cl, F, $CH_3$, COOH, $CONHCH_2Ph$ and $CONHCH_2$-pyrazinyl-$CH_3$, which substituents are completely different compared with the dihydropteridinones claimed according to the present invention.

Further, WO 2006/018185 A2 and WO 2006/018182 are related to the use of dihydropteridinones in the cancer therapy, the structure being different from the compounds as claimed.
The resistance of many types of tumours calls for the development of new pharmaceutical compositions for combating tumours. The object of the present invention is therefore to develop new compounds with antiinflammatory and antiproliferative activity, particularly to provide compounds for the treatment of various cancer diseases.

### Detailed description of the invention

Surprisingly it has been found that compounds of general formula (12) wherein the groups $R^1$ to $R^5$ and L, m, and n have the meanings given hereinafter act as inhibitors of specific cell cycle kinases. Thus, the compounds according to the invention may be used for example to treat diseases associated with the activity of specific cell cycle kinases and characterised by excessive or abnormal cell proliferation.

The present invention therefore relates to compounds of general formula (12)

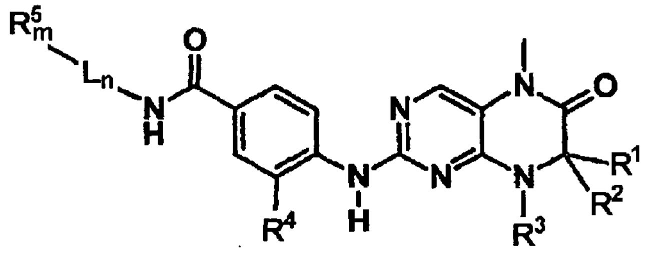

wherein

$R^1$ denotes hydrogen or $C_1$-$C_6$-alkyl, optionally substituted with one or more halogens,

$R^2$ denotes an -OH group, or

$R^3$ denotes a group selected from among $C_1$-$C_5$-alkyl and $C_3$-$C_7$-cycloalkyl,

$R^4$ denotes a group selected from among hydrogen, OMe, OH, Me, Et, Pr, OEt, NHMe, $NH_2$, F, CL, Br, O-propargyl, O-butynyl, CN, SMe, $NMe_2$, $CONH_2$, ethynyl, propynyl, butynyl and allyl,

L denotes a linker selected from among phenyl, phenylmethyl, cyclohexyl and optionally branched $C_1$-$C_6$-alkyl,

n denotes 0 or 1

m denotes 1 or 2

$R^5$ denotes a group selected from piperidinyl, and piperazinyl the heteroatoms of the groups of $R^5$ may be optionally substituted with $R^8$ and/or $R^9$,

$R^8$, $R^9$ denote unsubstituted substituents at the heteroatoms of the groups of $R^5$, which may be identical or different, and denote either hydrogen or a group selected from among $C_1$-$C_6$-alkyl, $C_1$-$C_4$-alkyl-$C_3$-$C_{10}$-cycloalkyl and $C_3$-$C_{10}$-cycloalkyl,
optionally in the form of the tautomers, the racemates, the enantiomers, the diastereomers and the mixtures thereof, and optionally the salts thereof.

Further, the invention discloses compounds of general formula (12) wherein

$R^1$ denotes hydrogen or optionally substituted $C_1$-$C_6$-alkyl,

$R^2$ denotes an O-X or S-X group, wherein X is selected from among hydrogen, optionally s
ubstituted $C_1$-$C_{12}$-alkyl, $C_2$-$C_{12}$-alkenyl, $C_2$-$C_{12}$-alkynyl, amid, halogen, $C_6$-$C_{14}$-aryl, heteroaryl, or
a group selected from among optionally substituted and/or bridged $C_3$-$C_{12}$-cycloalkyl, $C_3$-$C_{12}$-cycloalkenyl, $C_7$-$C_{12}$-polycycloalkyl, $C_7$-$C_{12}$-polycycloalkenyl, $C_5$-$C_{12}$-spirocycloalkyl, $C_3$-$C_{12}$-heterocycloalkyl which contains 1 to 2 heteroatoms, and $C_3$-$C_{12}$-heterocycloalkenyl which contains 1 to 2 heteroatoms, or

$R^1$ and $R^2$ together denote an optionally substituted $C_2$-$C_{12}$-alkenyl group, the double bond in the optionally substituted $C_2$-$C_{12}$-alkenyl group being preferably located as direct bond to the adjacent ring system,

$R^3$ denotes hydrogen or a group selected from among optionally substituted $C_1$-$C_{12}$-alkyl, $C_2$-$C_{12}$-alkenyl, $C_2$-$C_{12}$-alkynyl and $C_6$-$C_{14}$-aryl, or
a group selected from among optionally substituted and/or bridged $C_3$-$C_{12}$-cycloalkyl, $C_3$-$C_{12}$-cycloalkenyl, $C_7$-$C_{12}$-polycycloalkyl, $C_7$-$C_{12}$-polycycloalkenyl, $C_5$-$C_{12}$-spirocycloalkyl, $C_3$-$C_{12}$-heterocycloalkyl which contains 1 to 2 heteroatoms, and $C_3$-$C_{12}$-heterocycloalkenyl which contains 1 to 2 heteroatoms, or

$R^1$ and $R^3$ together denote a saturated or unsaturated $C_3$-$C_4$-alkyl bridge which may contain 1 heteroatom,

$R^4$ denotes hydrogen or a group selected from among -CN, hydroxy, $-NR^6R^7$ and halogen, or

a group selected from among optionally substituted $C_1$-$C_6$-alkyl, $C_2$-$C_6$-alkenyl, $C_2$-$C_6$-alkynyl, $C_1$-$C_5$-alkyloxy, $C_2$-$C_5$-alkenyloxy, $C_2$-$C_5$-alkynyloxy, $C_1$-$C_6$-alkylthio, $C_1$-$C_6$-alkylsulphoxo and $C_1$-$C_6$-alkylsulphonyl,

L denotes a linker selected from among optionally substituted $C_2$-$C_{10}$-alkyl, $C_2$-$C_{10}$-alkenyl, $C_6$-$C_{14}$-aryl, $C_2$-$C_4$-alkyl-$C_6$-$C_{14}$-aryl, $C_6$-$C_{14}$-aryl-$C_1$-$C_4$-alkyl, optionally bridged $C_3$-$C_{12}$-cycloalkyl and heteroaryl which contains 1 or 2 nitrogen atoms,

n denotes 0 or 1

m denotes 1 or 2

$R^5$ denotes a group selected from among optionally substituted morpholinyl, piperidinyl, piperazinyl, piperazinylcarbonyl, pyrrolidinyl, tropenyl, diketomethylpiperazinyl, sulphoxomorpholinyl, sulphonylmorpholinyl, thiomorpholinyl, -$NR^8R^9$ and azacycloheptyl, the heteroatoms of the groups of $R^5$ may be optionally substituted with $R^8$ and/or $R^9$,

$R^6$, $R^7$ which may be identical or different, denote hydrogen or $C_1$-$C_4$-alkyl, and

$R^8$, $R^9$ denote unsubstituted substituents at the heteroatoms of the groups of $R^5$, which may be identical or different, and denote either hydrogen or a group selected from among $C_1$-$C_6$-alkyl, $C_1$-$C_4$-alkyl-$C_3$-$C_{10}$-cycloalkyl, $C_3$-$C_{10}$-cycloalkyl, $C_6$-$C_{14}$-aryl, $C_1$-$C_4$-alkyl-$C_6$-$C_{14}$-aryl, pyranyl, pyridinyl, pyrimidinyl, $C_1$-$C_4$-alkyloxycarbonyl, $C_6$-$C_{14}$-arylcarbonyl, $C_1$-$C_4$-alkylcarbonyl, $C_6$-$C_{14}$-arylmethyloxycarbonyl, $C_6$-$C_{14}$-arylsulphonyl, $C_1$-$C_4$-alkylsulphonyl and $C_6$-$C_{14}$-aryl-$C_1$-$C_4$-alkylsulphonyl,

optionally in the form of the hydrates, tautomers, the racemates, the enantiomers, the diastereomers and the mixtures thereof, and optionally the salts thereof.

Further disclosed compounds of formula (12) are those wherein $R^1$, $R^2$, $R^3$, $R^4$, $R^6$ and $R^7$ are as hereinbefore defined, and

L denotes a linker selected from among optionally substituted $C_2$-$C_{10}$-alkyl, $C_2$-$C_{10}$-alkenyl, $C_6$-$C_{14}$-aryl, $C_2$-$C_4$-alkyl-$C_6$-$C_{14}$-aryl, $C_6$-$C_{14}$-aryl-$C_1$-$C_4$-alkyl, optionally bridged $C_3$-$C_{12}$-cycloalkyl and heteroaryl which contains 1 or 2 nitrogen atoms,

n denotes 0 or 1

m denotes 1 or 2

$R^5$ denotes a group which is bound to L via a nitrogen atom, selected from among optionally substituted morpholinyl, piperidinyl, piperazinyl, pyrrolidinyl, tropenyl, diketomethylpiperazinyl, sulphoxomorpholinyl, sulphonylmorpholinyl, thiomorpholinyl, -$NR^8R^9$ and azacycloheptyl, the heteroatoms of the groups of $R^5$ may be optionally substituted with $R^8$ and/or $R^9$,

$R^8$, $R^9$ denote unsubstituted substituents at the heteroatoms of the groups of $R^5$, which may be identical or different, and denote hydrogen or a group selected from among $C_1$-$C_6$-alkyl, $C_1$-$C_4$-alkyl-$C_3$-$C_{10}$-cycloalkyl, $C_3$-$C_{10}$-cycloalkyl, $C_6$-$C_{14}$-aryl, $C_1$-$C_4$-alkyl-$C_6$-$C_{14}$-aryl, pyranyl, pyridinyl, pyrimidinyl, $C_1$-$C_4$-alkyloxycarbonyl, $C_6$-$C_{14}$-arylcarbonyl, $C_1$-$C_4$-alkylcarbonyl, $C_6$-$C_{14}$-arylmethyloxycarbonyl, $C_6$-$C_{14}$-arylsulphonyl, $C_1$-$C_4$-alkylsulphonyl and $C_6$-$C_{14}$-aryl-$C_1$-$C_4$-alkylsulphonyl,

optionally in the form of the hydrates, tautomers, the racemates, the enantiomers, the diastereomers and the mixtures thereof, and optionally the salts thereof.

Further disclosed are compounds of formula (12) wherein $R^1$, $R^2$, $R^3$, $R^4$, $R^6$, $R^7$, L, n, and m are as hereinbefore defined, and

$R^5$ denotes a group which is bound to L via a nitrogen atom, selected from among optionally substituted piperidinyl, piperazinyl, piperazinyl, pyrrolidinyl, piperazinylcarbonyl, tropenyl, morpholinyl, and azacycloheptyl, the heteroatoms of the groups of $R^5$ may be optionally substituted with $R^8$ and/or $R^9$,

$R^8$, $R^9$ denote unsubstituted substituents at the heteroatoms of the groups of $R^5$, which may be identical or different, and denote hydrogen or a group selected from among $C_1$-$C_6$-alkyl, $C_1$-$C_4$-alkyl-$C_3$-$C_{10}$-cycloalkyl, $C_3$-$C_{10}$-cycloalkyl, $C_6$-$C_{14}$-aryl, $C_1$-$C_4$-alkyl-$C_6$-$C_{14}$-aryl, pyranyl, pyridinyl, pyrimidinyl, $C_1$-$C_4$-alkyloxycarbonyl, $C_6$-$C_{14}$-arylcarbonyl, $C_1$-$C_4$-alkylcarbonyl, $C_6$-$C_{14}$-arylmethyloxycarbonyl, $C_6$-$C_{14}$-arylsulphonyl, $C_1$-$C_4$-alkylsulphonyl and $C_6$-$C_{14}$-aryl-$C_1$-$C_4$-alkylsulphonyl,

optionally in the form of the hydrates, tautomers, the racemates, the enantiomers, the diastereomers and the mixtures thereof, and optionally the salts thereof.

Further disclosed are compounds of formula (12) wherein L, m, n and $R^3$ to $R^9$ are as hereinbefore defined, and

$R^1$ and $R^2$ together denote an optionally substituted $C_2$-$C_8$-alkenyl group, the double bond in the optionally substituted $C_2$-$C_8$-alkenyl group being preferably located as direct bond to the adjacent ring system,

optionally in the form of the hydrates, tautomers, the racemates, the enantiomers, the diastereomers and the mixtures thereof, and optionally the salts thereof.

Further disclosed are compounds of formula (12) wherein $R^1$, $R^2$, $R^3$, $R^4$, $R^6$, and $R^7$ are as hereinbefore defined, and

L denotes a linker selected from among optionally substituted $C_2$-$C_6$-alkyl, $C_2$-$C_6$-alkenyl, $C_6$-$C_{12}$-aryl, $C_2$-$C_4$-alkyl-$C_6$-$C_{12}$-aryl, $C_6$-$C_{12}$-aryl-$C_1$-$C_4$-alkyl, and optionally bridged $C_3$-$C_{12}$-cycloalkyl,

n denotes 0 or 1

m denotes 1

$R^5$ denotes a group which is bound to L via a nitrogen atom, selected from among piperidinyl, piperazinyl, pyrrolidinyl, the heteroatoms of the groups of $R^5$ may be optionally substituted with $R^8$ and/or $R^9$, and

$R^8$, $R^9$ denote unsubstituted substituents at the heteroatoms of the groups of $R^5$, selected from among hydrogen, $C_1$-$C_6$-alkyl, $C_1$-$C_4$-alkyl-$C_3$-$C_{10}$-cycloalkyl, and $C_3$-$C_{10}$-cycloalkyl,

optionally in the form of the hydrates, tautomers, the racemates, the enantiomers, the diastereomers and the mixtures thereof, and optionally the salts thereof.

Further disclosed are compounds of formula (12), wherein $R^1$, $R^5$, $R^6$, $R^7$, $R^8$, L, n, and m are as hereinbefore defined, and

$R^2$ denotes an O-X or S-X group, wherein X is selected from among hydrogen, optionally substituted $C_1$-$C_6$-alkyl, and $C_2$-$C_{12}$-alkenyl,

$R^3$ denotes hydrogen or a group selected from among optionally substituted $C_1$-$C_{10}$-alkyl, $C_3$-$C_{10}$-cycloalkyl, $C_3$-$C_{10}$-heterocycloalkyl and $C_6$-$C_{14}$-aryl, or

$R^1$ and $R^3$ together denote a saturated or unsaturated $C_3$-$C_4$-alkyl bridge which may contain 1 to 2 heteroatoms,

$R^4$ denotes a group selected from among hydrogen, OMe, OH, Me, Et, Pr, OEt, NHMe, $NH_2$, F, CL, Br, O-propargyl, O-butynyl, CN, SMe, $NMe_2$, $CONH_2$, ethynyl, propynyl, butynyl and allyl,

optionally in the form of the hydrates, tautomers, the racemates, the enantiomers, the diastereomers and the mixtures thereof, and optionally the salts thereof.

Further disclosed are also compounds of formula (12), wherein $R^1$, $R^2$, $R^3$, $R^4$, $R^5$, $R^6$, $R^7$, $R^8$, $R^9$, n, and m are as hereinbefore defined, and

L denotes a linker selected from among optionally substituted phenyl, phenylmethyl, cyclohexyl and optionally branched $C_1$-$C_6$-alkyl.

The invention further relates to compounds of formula (12) according to the invention for use as pharmaceutical compositions, particularly pharmaceutical compositions with an antiproliferative activity.

The invention also relates to the use of a compound according to the invention for preparing a pharmaceutical composition for the treatment and/or prevention of cancer, infections, inflammatory and autoimmune diseases.

Of particular importance according to the invention are compounds according to the invention for use as pharmaceutical compositions with an antiproliferative activity, particularly for the treatment of diseases characterized by an abnormal cell proliferation in a human or non-human mammalian body by inhibition of polo-like kinases as mitotic regulators.

In a preferred embodiment the invention relates to the use of a compound of formula (12) according to the invention, wherein the polo-like kinase is PLK-1.

In another preferred embodiment the invention discloses the use of a compound of formula (12) according to the invention, wherein the disease is characterized by inappropriate cellular proliferation, migration, apoptosis or angiogenesis, preferably by inappropriate cellular proliferation. Inappropriate cell proliferation means cellular proliferation resulting from inappropriate cell growth, from excessive cell division, from cell division at an accelerated rate and/or from inappropriate cell survival.

In another preferred embodiment the invention relates to the use of a compound of formula (12) according to the invention, wherein the disease is cancer selected from the group consisting of carcinomas, sarcomas, melanomas, myelomas, hematological neoplasias, lymphomas and childhood cancers.

In another preferred embodiment the invention relates to the use according to the invention, wherein the hematological neoplasia is leukemia.

In another preferred embodiment the invention relates to the use according to the invention, wherein the disease is cancer selected from the group consisting of mixed tumours, undifferentiated tumours and metastases thereof.

In a further embodiment the invention relates to the use of a compound of formula (12) according to the invention, optionally in form of its hydrates, tautomers, racemates, enantiomers, diastereomers and the mixtures thereof and optionally in form of the pharmacologically acceptable salts, for the preparation of a pharmaceutical composition for the treatment of autoimmune disorders selected from the group consisting of amyloidosis, systemic lupus erythematosus, rheumatoid arthritis, Crohn's disease, multiple sclerosis, systemic sclerosis (scleroderma), mixed connective tissue disease, Sjögren's syndrome, ankylosing spondylitis, autoimmune vasculitis, Behcet's syndrome, psoriasis, autoimmune arthritis, sarcoidosis and diabetes mellitus.

In a further embodiment the invention relates to the use of a compound of formula (12) according to the invention, optionally in form of its hydrates, tautomers, racemates, enantiomers, diastereomers and the mixtures thereof and optionally in form of the pharmacologically acceptable salts, for the preparation of a pharmaceutical composition for the treatment of fungous diseases including but not limited to candidiasis, cryptococcosis, aspergillosis, mucormycosis, tinea, dermatophytosis, histoplasmosis, blastomycosis, coccidiosis, pneumocystis.

In a further embodiment the invention discloses the treatment of a disease characterized by abnormal cell proliferation in a human or non-human mammalian body comprising administering to the mammal a therapeutically effective amount of a compound of formula (12), optionally in form of its hydrates, tautomers, racemates, enantiomers, diastereomers and the mixtures thereof and optionally in form of the salts.

In a further embodiment the invention discloses the treatment of patients who suffer from one or more diseases cited above comprising administering to the patient a therapeutically effective amount of a compound of formula (12), optionally in form of its hydrates, tautomers, racemates, enantiomers, diastereomers and the mixtures thereof and optionally in form of the salts.

The invention also dicloses the treatment and/or prevention of cancer, infections, inflammatory and autoimmune diseases, characterised in that a patient is given an effective amount of a compound of formula (12).

It is also described the treatment of a disease characterized by abnormal cell proliferation in a human or non-human mammalian body by inhibition of polo-like kinases as mitotic regulators in a mammal that comprises regulating, modulating, binding or inhibiting PLK activity and/or overexpression of PLK or one of the other PLK isoforms, preferably PLK-1.

In a further embodiment the invention relates to the use of a compound of formula (12) according to the invention, optionally in form of its hydrates, tautomers, racemates, enantiomers, diastereomers and the mixtures thereof and optionally in form of the pharmacologically acceptable salts thereof, wherein the active ingredient is administered orally, enterically, transdermally, intravenously, peritoneally or by injection, preferably intravenously.

Within the meaning of the present invention, a compound of formula (12), optionally in form of its hydrates, tautomers, racemates, enantiomers, diastereomers and the mixtures thereof and optionally in form of the salts, inhibits the proliferation of various human tumour cell lines including but not limited to Saos-2, H4, MDA-MB-435S, MDA-MB453, MCF7, HeLa S3, HCT116, Colo 205, HT29, FaDu, HL-60, K-562, THP-1, HepG2, A549, NCI-H460, NCI-H520, GRANTA-519, Raji, Ramos, BRO, SKOV-3, BxPC-3, Mia CaPa-2, DU145, PC-3, NCI-N87, MES-SA, SK-UT-1B and A431.

The invention also relates to pharmaceutical preparations, containing as active substance one or more compounds of general formula (12) or the physiologically acceptable or pharmacologically acceptable salts thereof, solvates, hydrates, polymorphs, physiologically functional derivatives and prodrugs thereof, optionally combined with conventional excipients, diluents and/or carriers.

The detailed concept of use of the dihydropteridinone compounds may be derived from WO 2006/018185 A2.

The invention also discloses a process for preparing a compound of general formula (12), wherein the following 7 steps may be performed separately and the product of every step may be purified, respectively, or the obtained product may be used as such in the subsequent step so that the steps may be combined in a successive order.

In Step 1, the process according to the present invention provides a compound of formula (3),

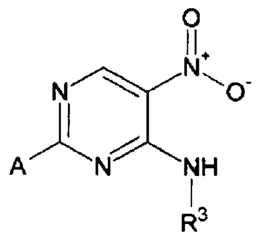

(3)

wherein

$R^3$ is as hereinbefore defined and A is a leaving group, characterised in that a compound of general formula (1)

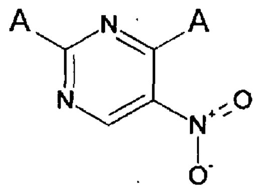

(1)

wherein
A is a leaving group,
is reacted with a compound of general formula (2)

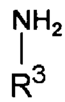

(2)

wherein
$R^3$ is as hereinbefore defined, to obtain a compound of general formula (3).

In Step 2, the process according to the present invention provides a compound of formula (4),

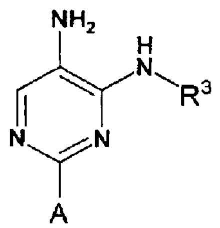

(4)

wherein

$R^3$ is as hereinbefore defined and A is a leaving group, characterised in that a compound of general formula (3)

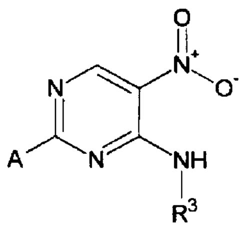

(3)

wherein
$R^3$ is as hereinbefore defined and A is a leaving group,
is hydrogenated at the nitro group in the presence of a suitable catalyst to obtain a compound of general formula (4).

In Step 3, the process according to the present invention provides a compound of formula (6),

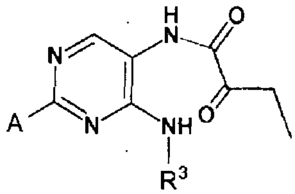

(6)

wherein

$R^3$ is as hereinbefore defined and A is a leaving group, characterised in that a compound of general formula (4)

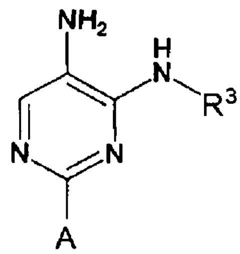

(4)

wherein
$R^3$ is as hereinbefore defined and A is a leaving group,
and an alpha-keto acid are reacted to form a compound of general formula (6).

In Step 4, the process according to the present invention provides a compound of formula (7) in form of the cis or trans compound or a mixture thereof,

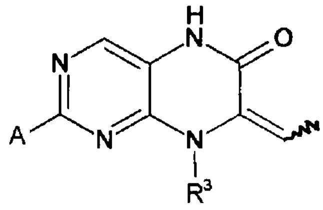

(7)

wherein

$R^3$ is as hereinbefore defined and A is a leaving group, characterised in that a compound of general formula (6)

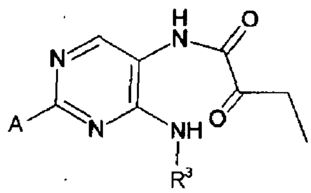

(6)

wherein
$R^3$ is as hereinbefore defined and A is a leaving group,
is cyclised to form the compound of formula (7) in form of the cis or trans compound or a mixture thereof.

In Step 5, the process according to the present invention provides a compound of formula (9) in form of the cis or trans compound or a mixture thereof,

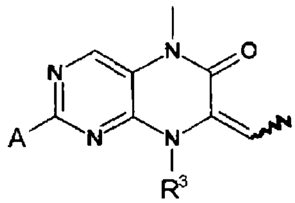

(9)

wherein

$R^3$ is as hereinbefore defined and A is a leaving group, characterised in that
a compound of general formula (7) in form of the cis or trans compound or a mixture thereof

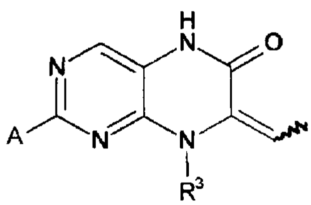

(7)

wherein
$R^3$ is as hereinbefore defined and A is a leaving group,
is reacted with a methylating reagent in the presence of a base to obtain the compound of formula (9) in form of the

cis or trans compound or a mixture thereof.

In Step 6, the process according to the present invention provides a compound of formula (11) in form of the cis or trans compound or a mixture thereof,

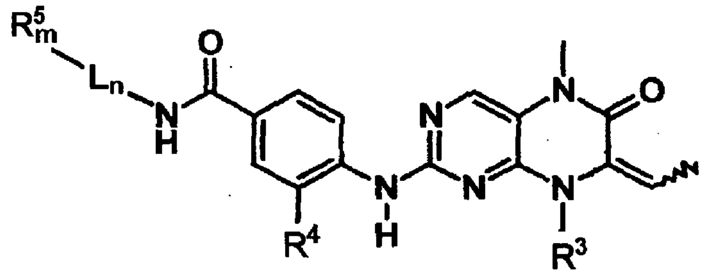

(11)

wherein

$R^3$, $R^4$, $R^5$, L, m, and n are as hereinbefore defined, characterised in that a compound of general formula (9) in form of the cis or trans compound or a mixture thereof

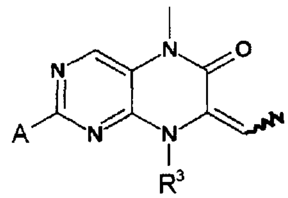

(9)

wherein
$R^3$ is as hereinbefore defined and A is a leaving group,
is reacted with an optionally substituted compound of general formula (10),

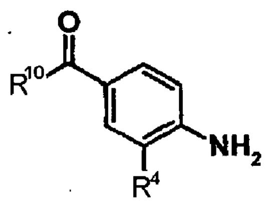

(10)

wherein
$R^4$ is as hereinbefore defined, and
$R^{10}$ is NH-$L_n$-$R^5_m$ and $R^5$, L, m and n are as hereinbefore defined.

In Step 7, the process according to the present invention provides a compound of formula (12),

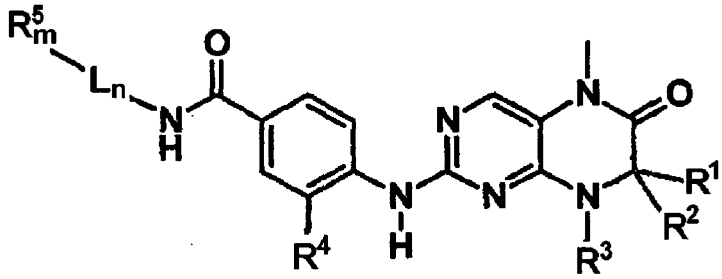

(12)

wherein

$R^1$ to $R^5$, L, m and n are as hereinbefore defined, characterised in that
a compound of general formula (11) in form of the cis or trans compound or a mixture thereof

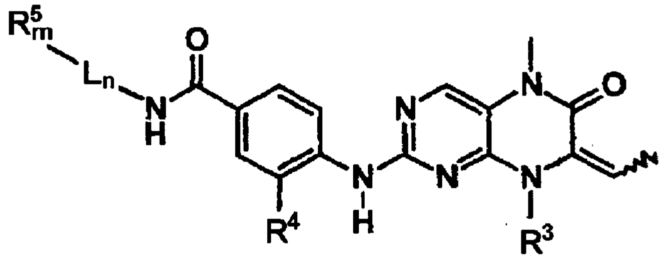

(11)

wherein
$R^3$ to $R^5$, L, m and n are as hereinbefore defined,
is reacted to form the compound of general formula (12) and the product is optionally subsequently purified according to known processes, preferably chromatographic processes.

**Definition of terms used**

The term "alkyl groups", including alkyl groups which are a part of other groups, denotes branched and unbranched alkyl groups with 1 to 12 carbon atoms, preferably 1 to 6 carbon atoms, most preferably 1 to 4 carbon atoms, such as, for example: methyl, ethyl, propyl, butyl, pentyl, hexyl, heptyl, octyl, nonyl, decyl and dodecyl.

Unless otherwise stated, the abovementioned terms propyl, butyl, pentyl, hexyl, heptyl, octyl, nonyl, decyl and dodecyl include all the possible isomeric forms. For example, the term propyl includes the two isomeric groups n-propyl and isopropyl, the term butyl includes n-butyl, iso-butyl, sec-butyl and tert-butyl, the term pentyl includes iso-pentyl, neopentyl, etc.

In the above-mentioned alkyl groups one or more hydrogen atoms may optionally be replaced by other groups. For example these alkyl groups may be substituted by halogen, preferably fluorine, chlorine and bromide. All the hydrogen atoms of the alkyl group may optionally be replaced.

The term "alkyl bridge", unless otherwise stated, denotes branched and unbranched alkyl groups with 1 to 5 carbon atoms, for example methylene, ethylene, propylene, isopropylene, n-butylene, iso-butyl, sec-butyl and tert-butyl etc. bridges. Methylene, ethylene, propylene and butylene bridges are particularly preferred. In the alkyl bridges mentioned 1 to 2 C-atoms may optionally be replaced by one or more heteroatoms selected from among oxygen, nitrogen or sulphur.

The term "alkenyl groups" including those which are a part of other groups denotes branched and unbranched alkylene groups with 2 to 10 carbon atoms, preferably 2 to 6 carbon atoms, most preferably 2 to 3 carbon atoms, provided that they have at least one double bond. Examples include: ethenyl, propenyl, butenyl, pentenyl etc. Unless otherwise stated, the above-mentioned terms propenyl, butenyl, etc. also include all the possible isomeric forms. For example, the term butenyl includes 1-butenyl, 2-butenyl, 3-butenyl, 1-methyl-1-propenyl, 1-methyl-2-propenyl, 2-methyl-1-propenyl, 2-methyl-2-propenyl and 1-ethyl-1-ethenyl.

In the above-mentioned alkenyl groups, unless otherwise stated, one or more hydrogen atoms may optionally be replaced by other groups. For example, these alkyl groups may be substituted by halogen, preferably fluorine, chlorine and bromide. All the hydrogen atoms of the alkenyl group may optionally be replaced.

The term "alkynyl groups" including those which are a part of other groups denotes branched and unbranched alkynyl groups with 2 to 10 carbon atoms, provided that they have at least one triple bond, for example ethynyl, propargyl, butynyl, pentynyl, hexynyl etc., preferably ethynyl or propynyl.

In the above-mentioned alkynyl groups, unless otherwise stated, one or more hydrogen atoms may optionally be replaced by other groups. For example, these alkyl groups may be substituted by halogen, preferably fluorine, chlorine or bromide. All the hydrogen atoms of the alkynyl group may optionally be replaced.

The term "aryl" denotes an aromatic ring system with 6 to 14 carbon atoms, preferably 6 to 10 carbon atoms, preferably phenyl, which, unless otherwise stated, may carry one or more of the following substituents, for example: OH, $NO_2$, CN, OMe, $-OCHF_2$, $-OCF_3$, $-NH_2$, halogen, for example fluorine or chlorine, $C_1$-$C_{10}$-alkyl, preferably $C_1$-$C_5$-alkyl, preferably $C_1$-$C_3$-alkyl, most preferably methyl or ethyl, -O-$C_1$-$C_3$-alkyl, preferably -O-methyl or-O-ethyl, -COOH, -COO-$C_1$-$C_4$-alkyl, preferably -O-methyl or-O-ethyl, $-CONH_2$.

Examples of "heteroaryl groups" wherein up to two carbon atoms are replaced by one or two nitrogen atoms include pyrrole, pyrazole, imidazole, triazole, pyridine, pyrimidine, while each of the above-mentioned heteroaryl rings may optionally also be anellated to a benzene ring, preferably benzimidazole. These heterocycles may optionally carry one or more of the following substituents, for example: F, Cl, Br, OH, OMe, methyl, ethyl, CN, $CONH_2$, $NH_2$, optionally substituted phenyl, optionally substituted heteroaryl, preferably optionally substituted pyridyl.

Examples of "cycloalkyl groups" are cycloalkyl groups with 3 to 12 carbon atoms, for example cyclopropyl, cyclobutyl, cyclopentyl, cyclohexyl, cycloheptyl or cyclooctyl, preferably cyclopropyl, cyclopentyl or cyclohexyl, while each of the above-mentioned cycloalkyl groups may optionally also carry one or more substituents, for example: OH, $NO_2$, CN, OMe, $-OCHF_2$, $-OCF_3$, $-NH_2$ or halogen, preferably fluorine or chlorine, $C_1$-$C_{10}$-alkyl, preferably $C_1$-$C_5$-alkyl, preferably $C_1$-$C_3$-alkyl, more preferably methyl or ethyl, -O-$C_1$-$C_3$-alkyl, preferably -O-methyl or -O-ethyl, -COOH, -COO-$C_1$-$C_4$-alkyl, preferably -COO-methyl or-COO-ethyl or-$CONH_2$. Particularly preferred substituents of the cycloalkyl groups are =O, OH, $NH_2$, methyl or F.

Examples of "cycloalkenyl groups" are cycloalkyl groups with 3 to 12 carbon atoms which have at least one double bond, for example cyclopropenyl, cyclobutenyl, cyclopentenyl, cyclohexenyl or cycloheptenyl, preferably cyclopropenyl, cyclopententyl or cyclohexenyl, while each of the above-mentioned cycloalkenyl groups may optionally also carry one or more substituents.

"= O" denotes an oxygen atom linked via a double bond.

Examples of "heterocycloalkyl groups", unless otherwise stated, include 3- to 12-membered, preferably 5-, 6- or 7-membered, saturated or unsaturated heterocycles which may contain as heteroatoms nitrogen, oxygen or sulphur, for example tetrahydrofuran, tetrahydrofuranone, gamma-butyrolactone, alpha-pyran, gamma-pyran, dioxolane, tetrahydropyran, dioxane, dihydrothiophene, thiolan, dithiolan, pyrroline, pyrrolidine, pyrazoline, pyrazolidine, imidazoline, imidazolidine, tetrazole, piperidine, pyridazine, pyrimidine, pyrazine, piperazine, triazine, tetrazine, morpholine, thiomorpholine, diazepan, oxazine, tetrahydro-oxazinyl, isothiazole, pyrazolidine, preferably morpholine, pyrrolidine, piperidine or piperazine, while the heterocyclic group may optionally carry substituents, for example $C_1$-$C_4$-alkyl, preferably methyl, ethyl or propyl.

Examples of "polycycloalkyl groups" are optionally substituted, bi-, tri-, tetra- or pentacyclic cycloalkyl groups, for example pinane, 2,2,2-octane, 2,2,1-heptane or adamantane.

Examples of "polycycloalkenyl groups" are optionally bridged and/or substituted preferably 8-membered bi-, tri-, tetra- or pentacyclic cycloalkenyl groups, preferably bicycloalkenyl or tricycloalkenyl groups, if they have at least one double bond, for example norbornene.

Examples of "spiroalkyl groups" are optionally substituted spirocyclic $C_3$-$C_{12}$ alkyl groups.

Generally, the term "halogen" denotes fluorine, chlorine, bromine or iodine, preferably fluorine, chlorine or bromine, most preferably chlorine.

The "leaving group A" denotes either identical or different leaving groups such as for example -O-methyl, -SCN, chlorine, bromine, iodine, methanesulphonyl, trifluoromethanesulphonyl or p-toluenesulphonyl, preferably chlorine.

## Preferred embodiments of the present Invention

The compounds according to the invention may be present in the form of the individual optical isomers, mixtures of the individual enantiomers, diastereomers or racemates, in the form of the hydrates, tautomers and also in the form of the free bases or the corresponding acid addition salts with pharmacologically acceptable acids - such as for example acid addition salts with hydrohalic acids, for example hydrochloric or hydrobromic acid, or organic acids, such as for example oxalic, fumaric, diglycolic or methanesulphonic acid. The compounds according to the invention may be also present in the form of solvates, hydrates, polymorphs, physiologically functional derivatives or prodrugs thereof.

The substituent $R^1$ may denote hydrogen or a group selected from among optionally substituted and/or branched $C_1$-$C_5$-alkyl, preferably methyl or ethyl.

The substituent $R^2$ may denote an O-X or S-X group, wherein X is selected from among hydrogen, optionally substituted and/or branched $C_1$-$C_5$-alkyl, and optionally substituted and/or branched $C_2$-$C_{10}$-alkenyl, preferably O-X, wherein X is hydrogen or optionally substituted and/or branched $C_1$-$C_5$-alkyl, most preferably hydrogen.

$R^1$ and $R^2$ together may denote an optionally substituted $C_2$-$C_6$-alkenyl group, the double bond in the optionally substituted $C_2$-$C_6$-alkenyl group being preferably located as direct bond to the adjacent ring system, preferably an optionally substituted $C_2$-$C_4$-alkenyl group, most preferably an optionally substituted $C_2$-$C_3$-alkenyl group, wherein the double bond is located to directly link the alkylene group and the adjacent ring system.

The substituent $R^3$ may denote hydrogen or a group selected from among optionally substituted and/or branched $C_1$-$C_{12}$-alkyl, preferably ethyl, propyl, butyl, pentyl or hexyl, more preferably propyl, butyl, pentyl or hexyl, $C_2$-$C_{12}$-alkenyl, preferably $C_5$-$C_8$-alkenyl, $C_2$-$C_{12}$-alkynyl, preferably $C_5$-$C_8$-alkynyl and $C_5$-$C_{14}$-aryl, preferably phenyl, a group selected from among optionally substituted and/or bridged $C_3$-$C_{12}$-cycloalkyl, preferably cyclopentyl or cyclohexyl, $C_3$-$C_{12}$-cycloalkenyl, preferably $C_5$-$C_8$-cycloalkenyl, $C_7$-$C_{12}$-polycycloalkyl, $C_7$-$C_{12}$-polycycloalkenyl, $C_5$-$C_{12}$-spirocycloalkyl, $C_3$-$C_{12}$-heterocycloalkyl, preferably pyranyl or piperinyl, pyrrolidinyl, pyrazinyl or morpholinyl which contains 1 to 2 heteroatoms, preferably oxygen or nitrogen, and $C_3$-$C_{12}$-heterocycloalkenyl which contains 1 to 2 heteroatoms, preferably oxygen or nitrogen.

Most preferably, the substituent $R^3$ denotes isopropyl, isobutyl, isopentyl, cyclopentyl, phenyl or cyclohexyl .

$R^1$ and $R^3$ together may denote a saturated or unsaturated $C_3$-$C_4$-alkyl bridge which may contain 1 heteroatom, preferably oxygen or nitrogen.

The substituent $R^4$ may denote hydrogen or a group selected from among -CN, hydroxy, -$NR^6R^7$ and halogen, preferably chlorine or fluorine, more preferably chlorine or a group selected from among optionally substituted $C_1$-$C_6$-alkyl, preferably methyl, ethyl or propyl, $C_2$-$C_6$-alkenyl, preferably ethenyl or propenyl, $C_2$-$C_6$-alkynyl, preferably ethynyl, propynyl or butynyl, $C_1$-$C_5$-alkyloxy, preferably methoxy, ethoxy or propargyloxy, $C_2$-$C_5$-alkenyloxy, $C_2$-$C_5$-alkynyloxy, $C_1$-$C_6$-alkylthio, $C_1$-$C_6$-alkylsulphoxo and $C_1$-$C_6$-alkylsulphonyl.

Most preferably, the substituent $R^4$ denotes methoxy, methyl, ethoxy, ethyl, propargyloxy or chlorine.

L may denote a linker selected from among optionally substituted and/or branched $C_2$-$C_6$-alkyl, preferably ethyl, propyl, butyl or pentyl, optionally substituted $C_2$-$C_6$-alkenyl, optionally substituted $C_6$-$C_{12}$-aryl, preferably phenyl, optionally substituted $C_2$-$C_4$-alkyl-$C_6$-$C_{12}$-aryl, optionally substituted $C_6$-$C_{12}$-aryl-$C_1$-$C_4$-alkyl, preferably phenylmethyl, optionally bridged and/or optionally substituted $C_3$-$C_{12}$-cycloalkyl, preferably cyclohexyl, and optionally substituted heteroaryl which contains 1 or 2 nitrogen atoms.

n denotes 0 or 1

m denotes 1 or 2, preferably 1.

$R^5$ may denote a group selected from among optionally substituted morpholinyl, piperidinyl, piperazinyl, piperazinylcarbonyl, pyrrolidinyl, tropenyl, diketomethylpiperazinyl, sulphoxomorpholinyl, sulphonylmorpholinyl, thiomorpholinyl, -$NR^8R^9$ and azacycloheptyl, preferably piperidinyl, morpholinyl, pyrrolidinyl, sulphoxomorpholiny, piperazinyl, thiomorpholinyl or tropenyl, more preferably piperidinyl, piperazinyl, pyrrolidinyl.

The heteroatoms of the groups of $R^5$ may be optionally substituted with $R^8$ and/or $R^9$.

$R^5$ preferably denotes a group which is bound to L via a nitrogen atom.

The groups $R^6$ and $R^7$ may be identical or different and may denote hydrogen or $C_1$-$C_4$-alkyl, preferably methyl or ethyl.

The groups $R^8$ and $R^9$ may be unsubstituted substituents at the heteroatoms of the groups of $R^5$, they may be identical or different and denote either hydrogen or a group selected from among $C_1$-$C_6$-alkyl, preferably methyl, ethyl or propyl, $C_1$-$C_4$-alkyl-$C_3$-$C_{10}$-cycloalkyl, preferably -$CH_2$-cyclopropyl, $C_3$-$C_{10}$-cycloalkyl, $C_6$-$C_{14}$-aryl, preferably phenyl,-$C_1$-$C_4$-alkyl-$C_6$-$C_{14}$-aryl, preferably benzyl, pyranyl, pyridinyl, pyrimidinyl, pyranyl, $C_1$-$C_4$-alkyloxycarbonyl, $C_6$-$C_{14}$-arylcarbonyl, $C_1$-$C_4$-alkylcarbonyl, $C_6$-$C_{14}$-arylmethyloxycarbonyl, $C_6$-$C_{14}$-arylsulphonyl, $C_1$-$C_4$-alkylsulphonyl and $C_6$-$C_{14}$-aryl-$C_1$-$C_4$-alkylsulphonyl.

More preferred, the substituents $R^8$ and $R^9$ may denote unsubstituted substituents at the heteroatoms of the groups of $R^5$, independently from each other selected from among hydrogen, $C_1$-$C_6$-alkyl, $C_1$-$C_4$-alkyl-$C_3$-$C_{10}$-cycloalkyl, and $C_3$-$C_{10}$-cycloalkyl.

Most preferably, the substituent $R^8$ denotes hydrogen, methyl, ethyl or propyl or -$CH_2$-cyclopropyl.

Most preferably, the substituent $R^9$ denotes hydrogen methyl, ethyl or propyl or -$CH_2$-cyclopropyl.

It may be advantageous if only one of $R^8$ and $R^9$ represents hydrogen and the other is selected to be different from hydrogen.

$R^{10}$ may be a substituent selected from NH-$L_nR^5_m$.

All the groups mentioned in the definition of $R^1$ to $R^{10}$ may optionally be branched and/or substituted.

The present application discloses the following compounds of formula (11):

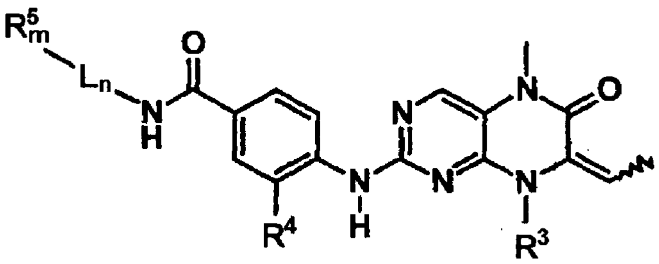

(11)

wherein $R^3$, $R^4$, $R^5$, L, n, and m are as hereinbefore defined, optionally in the form of the hydrates, tautomers, the racemates, the enantiomers, the diastereomers and the mixtures thereof, and optionally the salts thereof.

Also preferred are the following compounds falling under general formula (11):

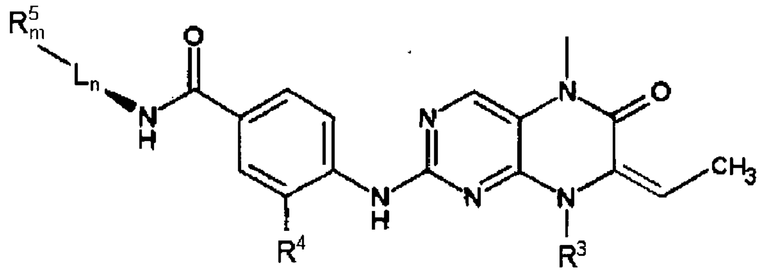

wherein $R^3$, $R^4$, $R^5$, L, m, and n are as hereinbefore defined, optionally in the form of the hydrates, tautomers, the racemates, the enantiomers, the diastereomers and the mixtures thereof, and optionally the salts thereof.

Particularly preferred are the following compounds falling under general formula (11):

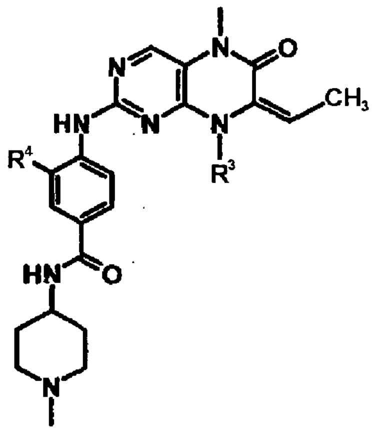

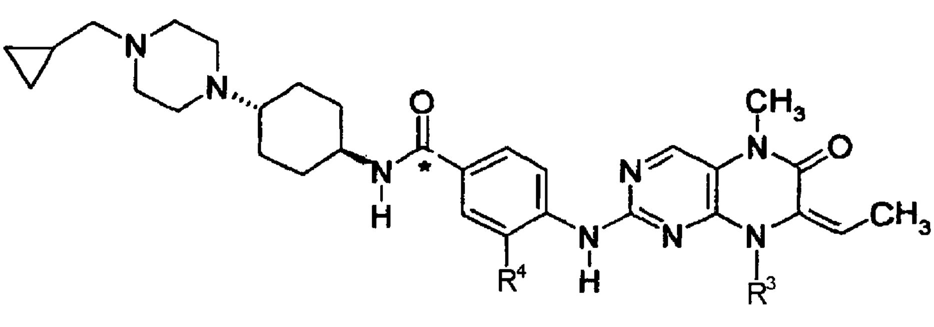

wherein $R^3$, and $R^4$ are as hereinbefore defined, optionally in the form of the hydrates, tautomers, the racemates, the enantiomers, the diastereomers and the mixtures thereof, and optionally the salts thereof.

Especially preferred are following compounds falling under general formula (11):

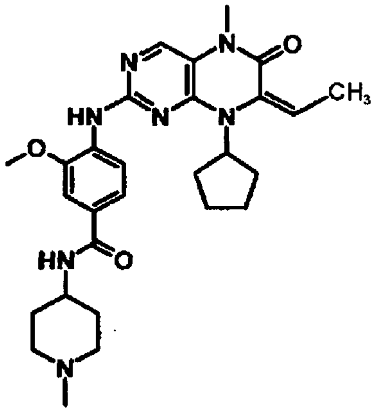

compound (11A)

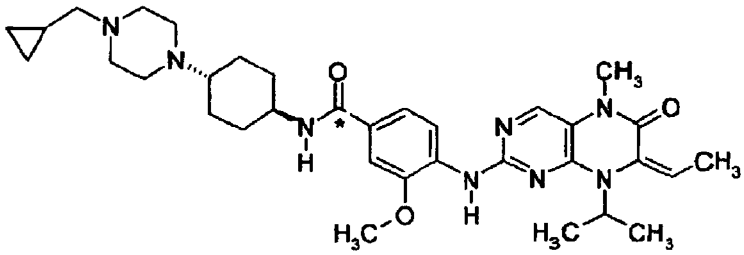

compound (11B)

The present invention is also directed to the following compounds of formula (12):

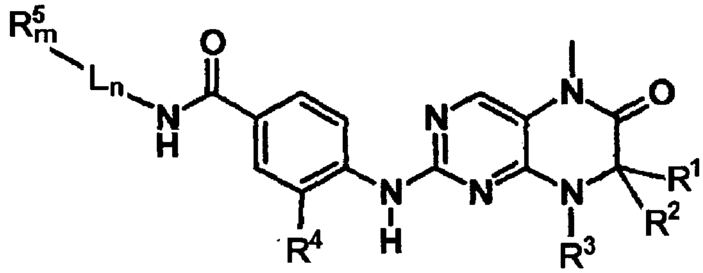

wherein $R^1$ to $R^5$, L, n, and m are as hereinbefore defined, optionally in the form of the hydrates, tautomers, the racemates, the enantiomers, the diastereomers and the mixtures thereof, and optionally the salts thereof.

Particularly preferred are the following compounds falling under general formula (12):

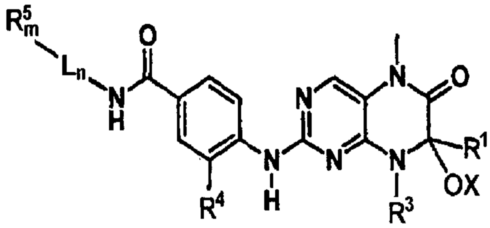

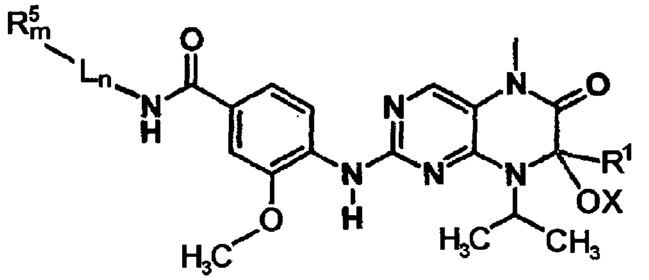

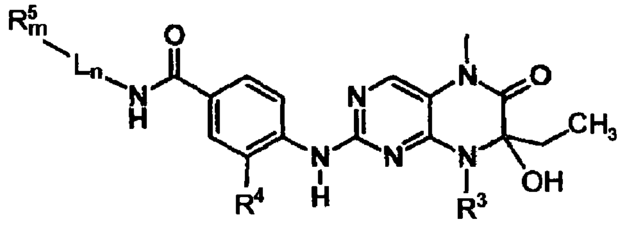

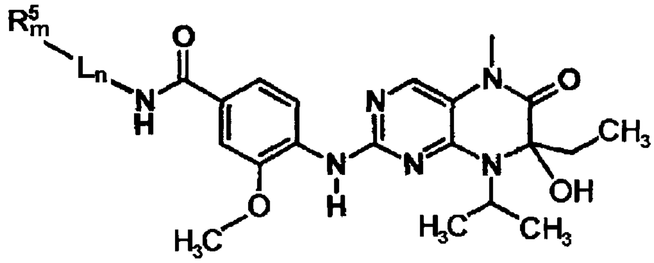

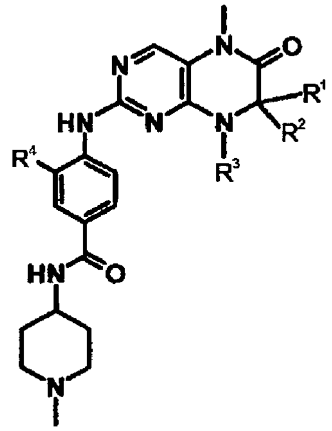

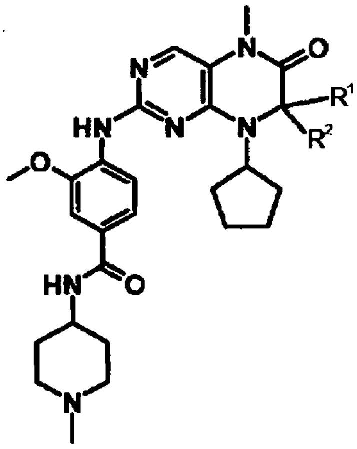

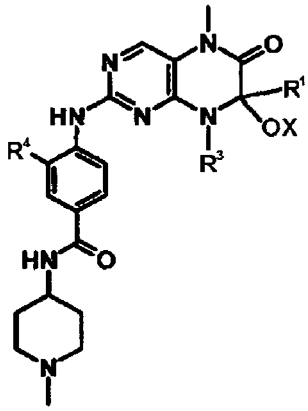

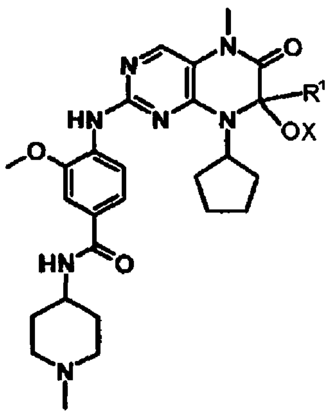

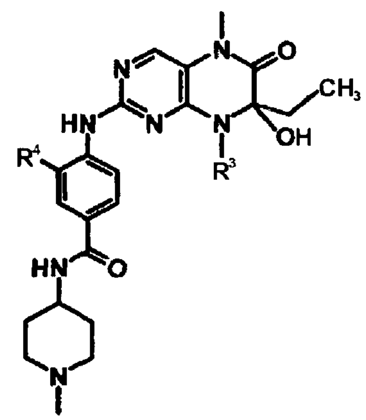

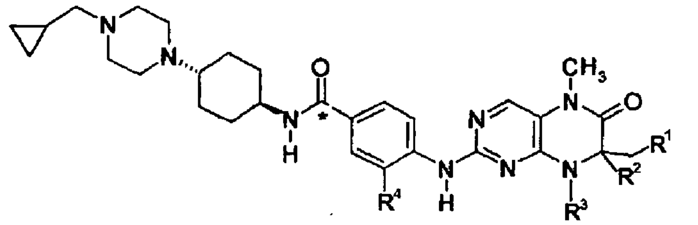

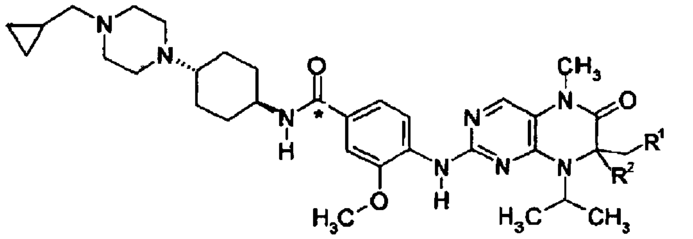

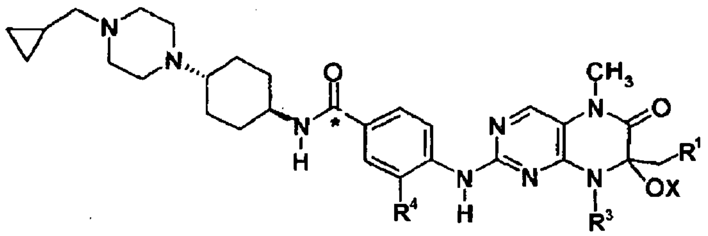

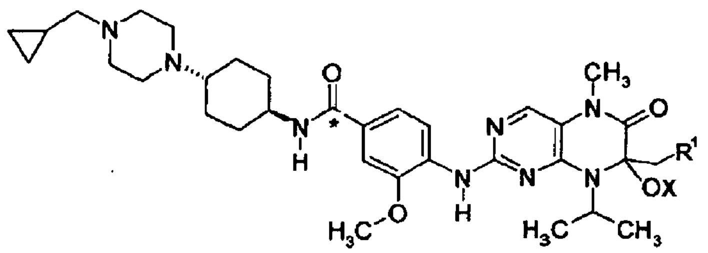

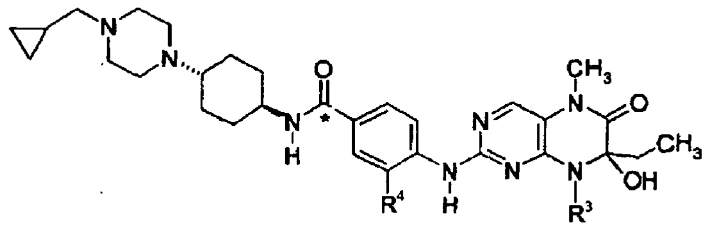

wherein $R^1$ to $R^5$, X, L, n, and m are as hereinbefore defined, optionally in the form of the hydrates, tautomers, the racemates, the enantiomers, the diastereomers and the mixtures thereof, and optionally the salts thereof.

Especially preferred are the following compounds falling under general formula (12):

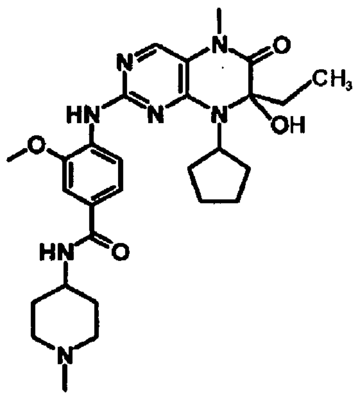

compound (12A)

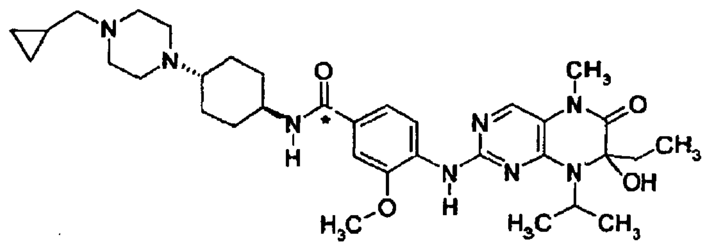

compound (12B)

optionally in the form of the hydrates, tautomers, the racemates, the enantiomers, the diastereomers and the mixtures thereof, and optionally the salts thereof.

The compounds according to the invention may be prepared by synthesis methods described hereinafter, while the substituents of general formulae (1) to (12) have the meanings given hereinbefore. This method is to be understood as an illustration of the invention.

**Method of Production**

Step 1

A compound of formula (1) is reacted with a compound of formula (2) to obtain a compound of formula (3) (see Diagram 1). This reaction may be carried out according to WO 00/43369 or WO 00/43372. Compound (1) is commercially available, for example, from City Chemical LLC, 139 Allings Crossing Road, West Haven, CT, 06516, USA. Also compound (2) is commercially available or may be prepared by procedures known from the literature.

**Diagram 1**

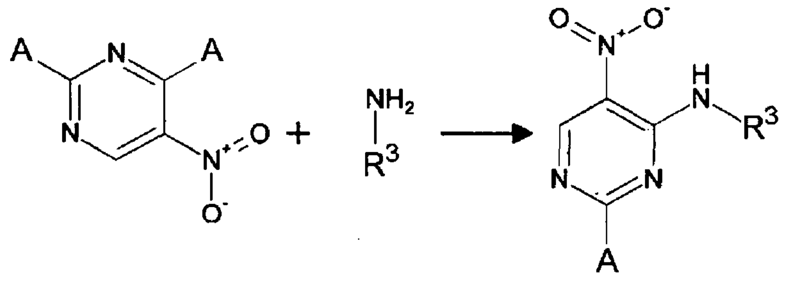

(1) (2) (3)

In Step 1, approximately 1 equivalent of the compound (1) and 1 to 1.5 equivalents, preferably 1.2 equivalents of a base, preferably potassium carbonate, potassium hydrogen carbonate, sodium carbonate or sodium hydrogen carbonate, calcium carbonate, most preferably potassium carbonate, are stirred in for example tetrahydrofuran, diethylether, cyclohexane, petroleum ether or dioxane, preferably cyclohexane or diethylether. At a temperature of 0 to 15°C, preferably 5 to 10°C, 1 equivalent of the amino compound of formula (2), optionally dissolved in an organic solvent, for example tetrahydrofuran, diethylether, cyclohexane or dioxane, is added dropwise. The reaction mixture is warmed to ambient temperature with stirring and then stirred for about 1 to 3 hours, preferably about 1 to 2 hours. Water is added to the reaction mixture followed by ethyl acetate. The organic phase is separated and the solvent evaporated under reduced pressure. The residue (compound 3) may be also used in Step 2 without any prior purification.

Step 2

The compound (3) obtained in Step 1 is hydrogenated at the nitro group to obtain compound (4) (see Diagram 2).

**Diagram 2**

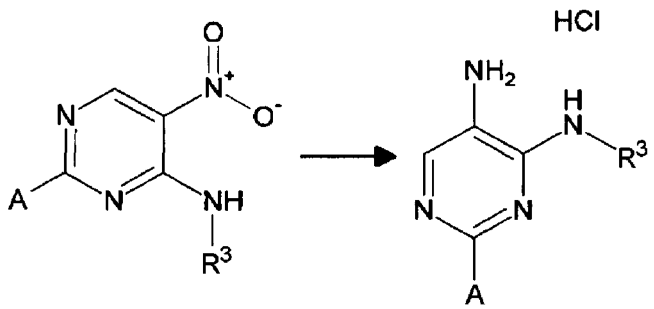

(3) (4)

In Step 2, 1 equivalent of the nitro compound (3) together with an inert solvent, e.g. tetrahydrofuran, diethylether, cyclohexane, petroleum ether or dioxane, preferably tetrahydrofuran, and a catalyst such as Pt/C in combination with vanadyl acetylacetonate or Raney nickel is hydrogenated under a hydrogen pressure between 30 and 50 psi (1 psi corresponds to 6894.8 pascal) at a temperature between 20 and 50°C. Then the catalyst is removed, the solvent distilled off and compound (4) is obtained which may be worked up as usual. Compound (4) may be purified by chromatography or by crystallisation or used as the crude product in Step 3 of the synthesis. It may be also useful to convert compound (4) into the hydrochloride salt thereof which may be used in Step (3) likewise.

Step 3

The compound (4) obtained in Step 2 is reacted with an alpha-keto acid (5) to obtain compound (6) (see Diagram 3).

## Diagram 3

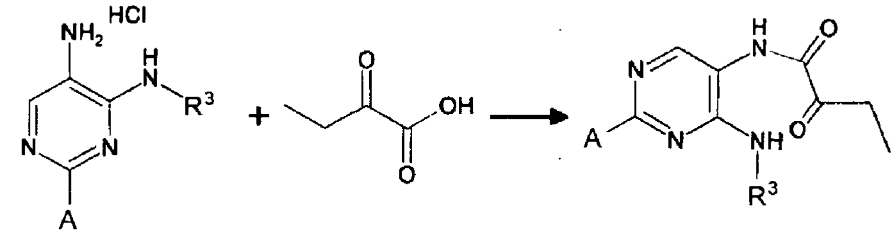

(4) (5) (6)

In Step 3, 1 equivalent of the compound (4) and 1 equivalent of an alpha-keto acid (5) is dissolved in an organic solvent such as tetrahydrofuran, N-methyl-2-pyrrolidone (NMP), diethylether, cyclohexane, petroleum ether or dioxane, preferably tetrahydrofuran or N-methyl-2-pyrrolidone under inert gas atmosphere and cooled down in an ice bath to a temperature between 0 and 15°C, preferably 5 to 10°C. Subsequently, about 1 equivalent of a carboxyl activating agent such as a carbodiimide, e.g. 1-ethyl-3-(3-dimethylaminopropyl) carbodiimide (EDC), usually in the form of the hydrochloride, is added while stirring. Then the ice bath is removed, stirring is continued overnight. The work up of compound (6) may be perfomed as usual. The organic phase may be separated, washed, dried and evaporated. The obtained compound (6) may be purified by chromatography or by crystallisation or used as crude product in Step 4 of the synthesis.

<u>Step 4</u>

The compound (6) obtained in Step 3 is cyclised to form the compound of formula (7) (see Diagram 4).

## Diagram 4

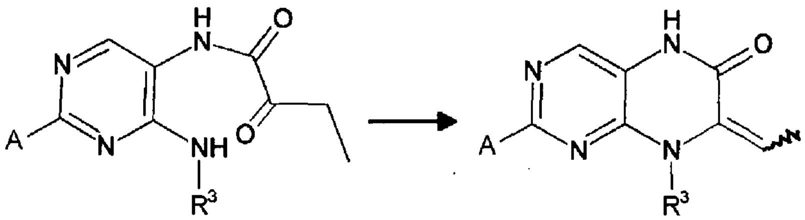

(6) (7)

In Step 4, 1 equivalent of compound (6) is solved in an organic solvent such as toluene, tetrahydrofuran, N-methyl-2-pyrrolidone (NMP), diethylether, cyclohexane, petroleum ether or dioxane, preferably toluene, and heated under reflux overnight while the formed water is separated. The obtained reaction mixture is cooled and the solvent is evaporated. Compound (7) is obtained in form of a mixture of the cis and trans compounds. The cis and trans compounds may be separated with known methods in synthetic organic chemistry or the mixture of the cis and trans compounds may be used as such in the next step without further purification.

<u>Step 5</u>

The compound (7) obtained in Step 4 may be alkylated as shown in Diagram 5 to obtain the compound of formula (9).

**Diagram 5**

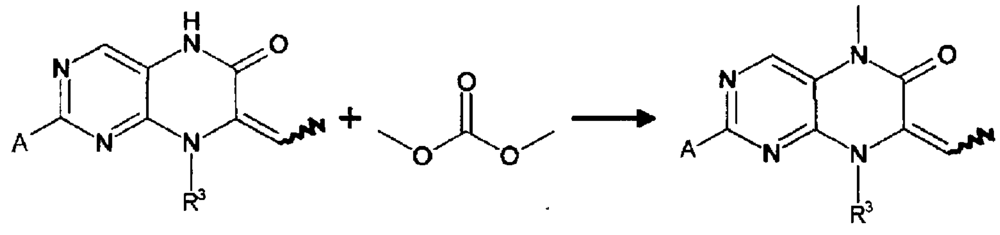

(7) (8) (9)

In Step 5, 1 equivalent of the amide of formula (7), a base, preferably potassium carbonate, potassium hydrogen carbonate, sodium carbonate or sodium hydrogen carbonate, calcium carbonate, most preferably potassium carbonate, and 10 to 15 equivalents of a methylating reagent (8), e.g. dimethyl carbonate are added. The reaction mixture is heated under stirring for several hours at about 100 to 150°C. The reaction mixture is poured into water. The organic phase is separated, washed, dried and evaporated. The obtained compound (9) may be purified by chromatography or by crystallisation or used as crude product in Step 6 of the synthesis. The obtained cis and trans compounds may be separated with known methods in synthetic organic chemistry or the mixture of the cis and trans compounds may be used as such in the next step without further purification.

Step 6

The amination of the compound (9) obtained in Step 5 to yield the compound of formula (11) (Diagram 6) may be carried out using the methods known from the literature.

**Diagram 6**

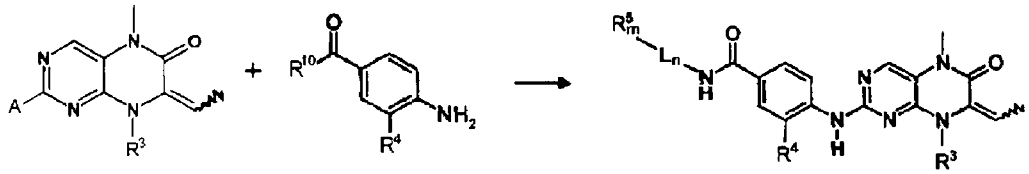

(9) (10) (11)

For example, 1 to 1.5 equivalents, preferably 1.1 equivalents of compound (9) and 1 equivalent of compound (10) are stirred with acid, for example 1 to 5 equivalents of p-toluenesulfonic acid and an high boiling secondary alkohol such as 4-methyl-2-pentanol, at reflux temperature for 1 to 48 hours, preferably about 5 hours. The precipitated product (11) is separated and worked up as ususal.. The obtained compound (11) may be purified by chromatography or by crystallisation or used as the crude product in Step 7 of the synthesis. The obtained cis and trans compounds may be separated with known methods in synthetic organic chemistry or the mixture of the cis and trans compounds may be used as such in the next step without further purification.

Step 7

The compound (11) obtained in Step 6 may be reacted to form the compound of formula (12'). In the present example the acid mediated hydratisation is described as shown in Diagram 7.

## Diagram 7

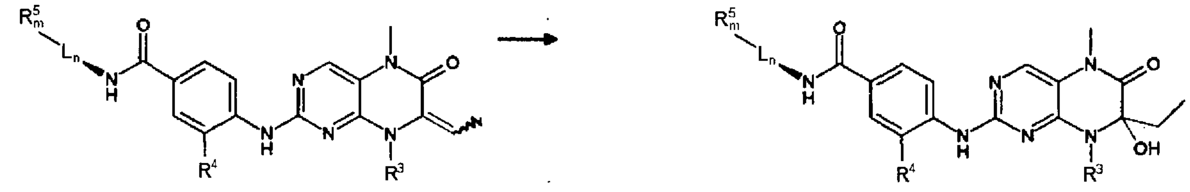

(11) (12')

In Step 7,1 equivalent of the compound of formula (11), and 3 to 5 equivalents of an acid, preferably hydrochloric, sulphuric or phosphoric acid, particularly preferred sulphuric acid, are stirred and heated under reflux for several hours. The reaction mixture obtained is made basic, diluted with water and worked up as usual. The obtained compound (12') may be purified according to well known methods in prior art such as chromatography, especially preparative HPLC.

### Administration and dosage forms

The compounds of general formula (12) may be used on their own or combined with other active substances according to the invention, optionally also in conjunction with other pharmacologically active substances.

Suitable preparations include for example tablets, capsules, suppositories, solutions, particularly solutions for injection (s.c., i.v., i.m.) and infusion, syrups, emulsions or dispersible powders. The amount of pharmaceutically active compound in each case should be in the range from 0.1 - 90 wt.%, preferably 0.5 - 50 wt.% of the total composition, i.e. in amounts which are sufficient to achieve the dosage range given below. The doses specified may, if necessary, be given several times a day.

Suitable tablets may be obtained, for example, by mixing the active substance(s) with known excipients, for example inert diluents such as calcium carbonate, calcium phosphate or lactose, disintegrants such as corn starch or alginic acid, binders such as starch or gelatine, lubricants such as magnesium stearate or talc and/or agents for delaying release, such as carboxymethyl cellulose, cellulose acetate phthalate, or polyvinyl acetate. The tablets may also comprise several layers.

Coated tablets may be prepared accordingly by coating cores produced analogously to the tablets with substances normally used for tablet coatings, for example collidone or shellac, gum arabic, talc, titanium dioxide or sugar. To achieve delayed release or prevent incompatibilities the core may also consist of a number of layers. Similarly the tablet coating may consist of a number of layers to achieve delayed release, possibly using the excipients mentioned above for the tablets.

Syrups or elixirs containing the active substances or combinations thereof according to the invention may additionally contain a sweetener such as saccharin, cyclamate, glycerol or sugar and a flavour enhancer, e.g. a flavouring such as vanillin or orange extract. They may also contain suspension adjuvants or thickeners such as sodium carboxymethyl cellulose, wetting agents such as, for example, condensation products of fatty alcohols with ethylene oxide, or preservatives such as p-hydroxybenzoates.

Solutions for injection and infusion are prepared in the usual way, e.g. with the addition of preservatives such as p-hydroxybenzoates, or stabilisers such as alkali metal salts of ethylenediamine tetraacetic acid, optionally using emulsifiers and/or dispersants, while if water is used as the diluent organic solvents may optionally be used as solubilisers or auxiliary solvents, and transferred into injection vials or ampoules or infusion bottles.

Capsules containing one or more active substances or combinations of active substances may for example be prepared by mixing the active substances with inert carriers such as lactose or sorbitol and packing them into gelatine capsules.

Suitable suppositories may be made for example by mixing with carriers provided for this purpose, such as neutral fats or polyethyleneglycol or the derivatives thereof.

Suitable excipients may be, for example, water, pharmaceutically acceptable organic solvents, such as paraffins (e.g. petroleum fractions), oils of vegetable origin (e.g. groundnut or sesame oil), mono- or polyfunctional alcohols (e.g. ethanol or glycerol), carriers such as e.g. natural mineral powders (e.g. kaolin, clays, talc, chalk), synthetic mineral powders (e.g. highly dispersed silica and silicates), sugar (e.g. glucose, lactose and dextrose), emulsifiers (e.g. lignin, spent sulphite liquors, methylcellulose, starch and polyvinylpyrrolidone) and lubricants (e.g. magnesium stearate, talc, stearic acid and sodium lauryl sulphate).

The preparations are administered in the usual way, preferably by oral or transdermal route, particularly preferably by oral route. When administered orally the tablets may, of course, contain additives, such as e.g. sodium citrate,

calcium carbonate and dicalcium phosphate together with various additives, such as starch, preferably potato starch, gelatine and the like, in addition to the abovementioned carriers. Lubricants such as magnesium stearate, sodium laurylsulphate and talc may also be used to form tablets. In the case of aqueous suspensions the active substances may be combined with various flavour enhancers or colourings in addition to the abovementioned excipients.

For parenteral use, solutions of the active substances may be prepared using suitable liquid carrier materials.

The dosage for intravenous use is 1 to 2000 mg per hour, preferably between 5 to 1000 mg per hour.

However, it may optionally be necessary to deviate from the amounts specified, depending on the body weight or method of administration, the individual response to the medication, the nature of the formulation used and the time or interval over which it is administered. Thus, in some cases, it may be sufficient to use less than the minimum quantity specified above, while in other cases the upper limit specified will have to be exceeded. When large amounts are administered it may be advisable to spread them over the day in a number of single doses.

**Biological properties**

As has been found, the compounds of general formula (12) are characterised by their wide range of applications in the therapeutic field. Particular mention should be made of those applications where the inhibition of specific cell cycle kinases, particularly the inhibiting effect on the proliferation of cultivated human tumour cells but also the proliferation of other cells, such as endothelial cells, for example, is involved.

As could be demonstrated by FACS analysis, the inhibition of proliferation brought about by the compounds according to the invention is mediated by the arrest of the cells, particularly at the G2/M phase of the cell cycle. The cells arrest, independently of the cells used, for a specific length of time in this phase of the cell cycle before programmed cell death is initiated. An arrest in the G2/M phase of the cell cycle is triggered, for example, by the inhibition of specific cell cycle kinases. Studies in model organisms such as *Schizosaccharomyces pombe* or *Xenopus,* or investigations in human cells have shown that the transition from the G2 phase to mitosis is regulated by the CDK1/cyclin B kinase (Nurse, 1990). This kinase, which is also known as the "mitosis promoting factor" (MPF), phosphorylates and thereby regulates a number of proteins, such as e.g. nuclear lamins, kinesin-like motor proteins, condensins and Golgi matrix proteins, which play an important part in the breakdown of the nuclear envelope, in centrosome separation, the formation of the mitotic spindle apparatus, chromosome condensation and the breakdown of the Golgi apparatus (Nigg. E., 2001). A murine cell line with a temperature-sensitive CDK1 kinase mutant shows a rapid breakdown of the CDK1 kinase and a subsequent arrest in the G2/M phase after a temperature increase (Th'ng et al., 1990). The treatment of human tumour cells with inhibitors against CDK1/cyclin B such as e.g. butyrolactone also leads to an arrest in the G2/M phase and subsequent apoptosis (Nishio, et al. 1996). Another kinase which is involved in the G2 and mitosis phase is polo-like kinase 1 (Plk1), which is responsible for the maturation of the centrosomes, for the activation of the phosphatase Cdc25C, as well as for the activation of the anaphase promoting complex (see Glover et al., Genes & Dev. 1998; 12: pp. 3777-3787, Qian et al. 2001, Mol Biol Cell. 12: 1791-9). The injection of Plk1 antibodies leads to a G2 arrest in untransformed cells whereas tumour cells arrest in the mitosis phase (Lane and Nigg, 1996). In addition, the protein kinase aurora B has been described as having an essential function during entry into mitosis. Aurora B phosphorylates histone H3 at Ser11 and thereby initiates chromosome condensation (Hsu, J.Y. et al., 2000). A specific cell cycle arrest in the G2/M phase may, however, also be triggered e.g. by the inhibition of specific phosphatases such as e.g. Cdc25C (Russell and Nurse, 1986). Yeasts with a defective cdc25 gene arrest in the G2 phase, while overexpression of cdc25 leads to early entry into the mitosis phase (Russell and Nurse, 1987). However, an arrest in the G2/M phase can also be triggered by the inhibition of certain motor proteins, so-called kinesins such as e.g. Eg5 (Mayer et al., 1999), or by agents which stabilise or destabilise microtubu les (e.g. colchicin, taxol, etoposide, vinblastin, vincristin) (Schiff and Horwitz, 1980).

In view of their biological properties the compounds of general formula (12) according to the invention, their isomers and their pharmacologically acceptable salts are suitable for the treatment of diseases characterised by excessive or abnormal cell proliferation.

Such diseases include, for example: viral infections (e.g. HIV and Kaposi's sarcoma); inflammatory and autoimmune diseases (e.g. colitis, arthritis, Alzheimer's disease, glomerulonephritis and wound healing); bacterial, fungal and/or parasitic infections; leukaemias, lymphoma and solid tumours; skin diseases (e.g. psoriasis); bone diseases; cardiovascular diseases (e.g. restenosis and hypertrophy). They are also suitable for protecting proliferating cells (e.g. hair, intestinal, blood and progenitor cells) from damage to their DNA caused by radiation, UV treatment and/or cytostatic treatment (Davis et al., 2001).

The inventive compounds may be used for the prevention, short-term or long-term treatment of the abovementioned diseases, also in combination with other active substances used for the same indications, e.g. cytostatics.

The activity of compounds according to the invention can be determined in a FACS analysis, for example on HeLaS3 cells. The compounds can also be evaluated in a hERg channel assay and the potency in kinase and cell proliferations assays in vitro can be examined. In the test methods, the tested compounds exhibited a good activity and/or potency as will be discussed in the following.

FACS analysis

Propidium iodide (PI) binds stoichiometrically to double-stranded DNA, and is thus suitable for determining the percentage of cells in the G1, S and G2/M phase of the cell cycle on the basis of the cell DNA content. Cells in the GO and G1 phase have a diploid DNA content (2N), whereas cells in G2 or mitosis have a 4N DNA content.

For PI staining, 0.4 million HeLaS3 cells are seeded, for example, on a 75 $cm^2$ cell culture flask, and after 24 h either 1 % DMSO is added as control or the substance is added in various concentrations (in 1% DMSO). The cells are incubated for 24 h with the substance or with DMSO, before the cells are washed with 2 x PBS and detached with trypsin /EDTA. The cells are centrifuged (1000 rpm, 5 min, 4°C), and the cell pellet is washed 2 x with PBS, before the cells are resuspended in 0.1 ml of PBS. Then the cells are fixed with 80% ethanol for 16 hours at 4°C or alternatively for 2 hours at - 20°C. The fixed cells ($10^6$ cells) are centrifuged (1000 rpm, 5 min, 4°C), washed with PBS and then centrifuged again. The cell pellet is resuspended in 2 ml of Triton X-100 in 0.25 % PBS, and incubated for 5 min on ice, before 5 ml of PBS are added and the mixture is centrifuged again. The cell pellet is resuspended in 350 μl of PI stain solution (0.1 mg/ml of RNase A, 10 μg/ml of prodium iodide in 1 x PBS). The cells are incubated for 20 min in the dark with the stain buffer before being transferred into sample measuring vessels for the FACS scan. The DNA measurement is carried out in a Becton Dickinson FACS Analyzer, with an argon laser (500 mW, emission 488 nm), and the DNA Cell Quest Program (BD). The logarithmic PI fluorescence is determined with a band-pass filter (BP 585/42). The cell populations in the individual phases of the cell cycle are quantified with the ModFit LT program of Becton Dickinson.

hERG channel assay

HEK (human embryonic kidney) 293 cells are stably transfected with hERG cDNA (obtained courtesy of M. Sanguinetti, University of Utah). Cells determined for use in patch clamp experiments are plated on glass coverslips in 24 well plates and are cultivated without antibiotic during this time.

Coverslips are placed at the bottom of a 2 mL perfusion chamber mounted on the stage of an inverted microscope. Cells are superfused with a bath solution containing (mM): NaCl (137), KCl (4.0), $MgCl_2$ (1.0), $CaCl_2$ (1.8), Glucose (10), HEPES (10), pH 7.4 with NaOH. Patch pipettes are made from borosilicate glass tubing (Hilgenberg, Malsfeld, FRG) using a horizontal puller (DMZ-Universal Puller, Zeitz-Instrumente, Martinsried, FRG) and filled with pipette solution containing (mM): K-aspartate (130), $MgCl_2$ (5.0), EGTA (5.0), $K_2ATP$ (4.0), HEPES (10.0), pH 7.2 with KOH. Resistance of the microelectrodes is in the range between 2 and 5 MΩ. Chemicals for the solutions are of analytical grade and are purchased from various commercial providers including SIGMA, ICN, and Merck KG.

Membrane currents are recorded using an EPC-10 or equivalent patch clamp amplifier (HEKA Electronics, Lambrecht, FRG) using the whole-cell configuration of the patch-clamp technique. hERG-mediated inactivating tail currents are elicited using a brief hyperpolarising pulse after channel inactivation at a depolarised potential.

Four concentrations of the compound of formula (12B) according to the present invention (0.3, 1, 3, 10 μM) are applied on different cells. A steady state level of baseline current is measured prior to the application of the test article.

The compound samples are dissolved in DMSO to yield a 10 mM stock solution which is diluted further in DMSO to stock solutions containing 3, 1, and 0.3 mM. Final dilutions in extracellular buffer are prepared freshly from these stocks by a 1:1000 dilution step each before starting the experiments

Peak current amplitudes are measured for each hyperpolarising step. For baseline and each concentration the peak currents of the three last sweeps before switch of perfusion are averaged. Residual currents (I/Io) are calculated for each cell as the fraction of actual average peak current and average baseline peak current. Current inhibition is expressed as $(1 - I/I_0) * 100\%$. Current inhibition for all cells is reported as mean ± SD. The logistic concentration-response curve of the following form is fitted to the residual current data:

$$I/I_0 = 1 - 1/(1+(C/IC_{50})^p)$$

C: actual concentration of compound (in μM)
$IC_{50}$ half-inhibitory concentration (in μM)
p Hill slope

Result: The residual currents of tested compound (12B) showed a value of 9.5 μM, which is a surprisingly high value and confirms the excellent characteristics and properties of the inventive compounds.

Potency in kinase and cell proliferations assays in vitro

The compound of formula (12B) according to the present invention is tested for its pharmacodynamic activity in kinase assays and cell proliferation assays in vitro as discussed in the following.

The serine/threonine kinase Plk1 performs a crucial function in cell-cycle progression and is a key regulator of multiple steps in mitosis as already discussed (see Glover et al., Genes & Dev. 1998; 12: pp. 3777-3787, Nigg et al., Curr. Opin. Cell Biol. 1998; 10: pp. 776-783, and Donaldson et al., J Cell Sci. 2001; 114: pp. 2357-2358). It has been implicated in the regulation of CDK1 at mitotic entry, centrosome maturation and separation, bipolar spindle formation, chromosome segregation, regulation of the anaphase-promoting complex and cytokinesis. Since Plk1 is active in mitosis of all dividing cells, it is likely that Plk1 inhibition will suppress tumour growth in many human cancer types, regardless of their organ derivation or oncogene and tumour suppressor gene status. Overexpression of Plk1 has been documented for various tumour types such as non-small cell lung cancer, squamous cell carcinomas, breast carcinomas or colorectal cancers (cf. Wolf et al., Oncogene 1997; 14: pp. 543-549, Knecht et al., Cancer Res. 1999; 59: pp. 2794-2797, Wolf et al., Pathol Res Pract. 2000, 196: pp. 753-759, and Takahashi et al., Cancer Sci. 2003; 94: pp. 148-152) and serves as a prognostic marker in certain cancer types.

The aim of the present study is to assess the inhibitory effect the compound of formula (12B) on the kinase activity of the Plk1 enzyme. In addition, the inhibitory effect of this compound on the proliferation rate of NCI-H460 human non-small cell lung cancer cells and human HCT 116 colon carcinoma cells is assessed.

Kinase assays are performed using full-length recombinant Plk1 enzyme produced in a baculovirus expression system and casein from bovine milk as a substrate. Results represent the means of 2 independent experiments with each data point measured in duplicate. NCI-H460 human non-small cell lung cancer and HCT 116 human colon carcinoma cell lines are used in Alamar Blue™ based proliferation assays. Results represent the means of 3 dose titrations for each cell line with each data point measured in duplicate. Two independent kinase assay experiments are carried out to determine the inhibitory activity of compound (12B) on the Plk1 enzyme. 7 concentrations of compound (12B) are used and each concentration is tested in duplicate measurements.

For each cell line a proliferation assay experiment is carried out to determine the $EC_{50}$ values in triplicate. 12 concentrations of each compound are used and each concentration is tested in duplicate measurements.

The compound is dissolved in DMSO at a concentration of 10 mM. For the proliferation assay and the first kinase assay the compound is freshly dissolved. For the second kinase assay a 10 mM stock solution in DMSO is used.

A baculoviral expression system is used to generate full-length recombinant Plk1 enzyme for the assay. Casein from bovine milk is used as a substrate. All other reagents are from the highest grade of purity commercially available.

Plk1 low enzyme inhibition assay

The full-length coding region of the human Plk1 enzyme (residues 1-603) is subcloned into a gluthathione-S-transferase (GST) baculoviral expression vector (pAcG2T; BD Biosciences) and active enzyme with a N-terminal GST-tag (GST-Plk1) is expressed in Sf21 insect cells. Cells are incubated for 3 days with recombinant baculovirus stock and 3 hours prior to harvest ocadaic acid (final concentration 0.1 μM, Calbiochem) is added to the cells. GST-Plk1 is extracted from the cells after lysis in buffer A (50 mM HEPES pH 7.5, 10 mM $MgCl_2$, 1 mM DTT, 5 μg/ml leupeptin, 5 μg/ml aprotinin, 100 μM NaF, 100 μM PMSF, 10 mM ß-glycerolphosphate, 0.1 mM $Na_3VO_4$, 30 mM 4-nitrophenyl-phosphate) by incubating the lysate with glutathione sepharose beads and eluting the enzyme with buffer B (100 mM TRIS/HCl pH 8, 120 mM NaCl, 20 mM reduced glutathione, 10 mM $MgCl_2$, 1 mM DTT).

Enzyme activity assays are performed in the absence or presence of serially diluted inhibitor. Casein from bovine milk (Sigma) dissolved in buffer C (15 mM $MgCl_2$, 25 mM MOPS pH 7.0, 1 mM DTT, 1.7 mM EGTA, 20 mM ß-glycerolphosphate) is used as substrate. As a negative control the incubation is performed in the absence of the kinase. As a positive control the reaction is performed in the absence of any test compound. The kinase assay is performed in a 96-well polystyrene microtiter plate formate in a final volume of 60 μl containing a final concentration of 7.5 μM ATP, 1% DMSO (v/v), 10 μg of casein as substrate, approx. 0.02 μg of virally expressed and purified GST-Plk1 protein and diluted test compound (in the range of 10 μM to 10 μM). The test compound (10 mM stock solutions in DMSO) is first diluted 1:10 in DMSO. Further dilutions of the test compound is made in assay buffer (15 mM $MgCl_2$, 25 mM MOPS pH 7.0, 1 mM DTT) containing 0.5 mg/ml casein and DMSO (to yield a final assay concentration of 1%). 10 μl of different dilutions of the compound (in assay buffer containing 0.5 mg/ml casein and 6% DMSO), 20 μl of additional casein (0.25 mg/ml), and 20 μl of GST-Plk1 (0.02 μl eluate in 20 μl of assay buffer) are mixed. The reaction is initiated by the addition of 10 μl ATP-mix (45 μl 1 mM adenosine-5'triphosphate in assay buffer mixed with 30 μCi -γ-$^{33}$P-ATP and adjusted to a final volume of 1 ml in assay buffer) and incubated for 45 min at 30°C and 650 rpm. After incubation, plates are put on ice, and proteins are precipitated by the addition of 125 μl of ice-cold 5% TCA per well. After 15 min on ice the precipitates are transferred to MultiScreen™ mixed ester cellulose filter plates (Millipore), collected by vacuum filtration and washed 4 x with 250 μl 1% TCA (room temperature). The filter plates are dried at 60°C, then 25 μl of Ultima Gold™

scintillation liquid (Packard) is added per well, the plate is sealed with tape and counted after 1 h in a Micro-Beta Scintillation counter (Wallac). All data points are measured in duplicates.

Data are fitted by iterative calculations using a sigmoidal curve analysis program (Graph Pad Prism version 3.03) with variable Hill slope.

Potency on PLK1

It could be shown that compound (12B) inhibited Plk1 as indicated in the following Table 1.

Table 1 Compound (12B) in the Plk1 kinase assay

| Compound | Experiment number | $IC_{50}$ [nM] | Mean $IC_{50}$ [nM] |
|---|---|---|---|
| compound 12B | 1 | 8.1 | 8.0 |
| compound 12B | 2 | 7.9 | |

Proliferation assays:

HCT 116 human colorectal carcinoma (ATCC CCL 247) and NCI-H460 human lung carcinoma (ATCC HTB-177) cells are used in an Alamar Blue™ based proliferation assay.

Alamar Blue™ cell proliferation assay

The Alamar Blue™ assay is designed to quantitatively measure the proliferation of cells by incorporating a fluorometric/colorimetric growth indicator that is based on the detection of metabolic activity in viable cells.

HCT 116 and NCI-H460 cells are grown in IMDM medium containing 10 % FCS and 2 mM L-glutamine. They are maintained at 37°C and 5 % $CO_2$ in a humidified atmosphere with a split ratio of 1:5. For proliferation assays in the 96-well format, penicillin-streptomycin is added to the medium at a final concentration of 100 u/ml penicillin and 100 μg/ml streptomycin.

On day one of the experiment, 1000 cells in 180 μl medium are seeded into each well of a sterile 96-well flat-bottom plate. The plates are kept in the incubator overnight. The compounds are dissolved in DMSO at a concentration of 1000 μM. On day two of the experiment, 12 serial dilutions (1:3) of the compounds are prepared in medium containing 0.1 % DMSO. 20 μl of the dilutions are added to each well to yield a final volume of 200 μl per well. As a control, medium containing 0.1% DMSO is added to the designated wells. The cells are then incubated for 72 h. After this incubation period, 20 μl of Alamar Blue™ is added to each well. After 6 h of incubation, the plates are measured in a fluorescence spectrophotometer (excitation 531 nm, emission 595 nm, slits 15, integrate time 0.1). All data points are measured in duplicates.

The mean value from the duplicates is taken and the background is subtracted. The "0.1% DMSO value" (the mean value obtained from wells containing cells plus 0.1% DMSO containing medium) is taken as "100 % control". The data are fitted by iterative calculations using a sigmoidal curve analysis program (Smiley - Graph Pad Prism based program) with variable Hill slope.

Efficacy in proliferation assays NCl-H460 and HCT 116)

The results are summarized in the following Tables.

Table 2 Activity of compound (12B) in cell proliferation assays (HCT 116 cells)

| | Measurement 1 $EC_{50}$ [nM] | Measurement 2 $EC_{50}$ [nM] | Measurement 3 $EC_{50}$ [nM] | Mean $EC_{50}$ [nM] |
|---|---|---|---|---|
| compound 12B | 311 | 335 | 174 | 273 |

Table 3 Activity of compound (12B) in cell proliferation assays (NCI-H460 cells)

| | **Measurement I $EC_{50}$ [nM]** | **Measurement 2 $EC_{50}$ [nM]** | **Measurement 3 $EC_{50}$ [nM]** | **Mean $EC_{50}$ [nM]** |
|---|---|---|---|---|
| compound 12B | 517 | 471 | 302 | 430 |

In summary, the compound of formula (12B) inhibited Plk1 kinase activity with $IC_{50}$ of 8 nM. The tested compound of formula (12B) shows potent activity in the enzymatic assay and also potency in cellular assays. Potentially relevant in vivo activity will depend on the pharmacokinetics and biodistribution of this compound. The compound of formula (12B) inhibed proliferation of HCT 116 and NCI-H460 cells with $EC_{50}$ values of 273 and 430 nM. As a result, the compound of formula (12B) inhibits Plk1 kinase activity and inhibits cell proliferation.

The formulation examples that follow illustrate the present invention:

**Examples of pharmaceutical formulations:**

A) Tablets

| | per tablet |
|---|---|
| active substance | 100 mg |
| lactose | 140 mg |
| corn starch | 240 mg |
| polyvinylpyrrolidone | 15 mg |
| magnesium stearate | 5 mg |
| | |
| total | 500 mg |

The finely ground active substance, lactose and some of the corn starch are mixed together. The mixture is screened, then moistened with a solution of polyvinylpyrrolidone in water, kneaded, wet-granulated and dried. The granules, the remaining corn starch and the magnesium stearate are screened and mixed together. The mixture is compressed to produce tablets of suitable shape and size.

B) Tablets

| | per tablet |
|---|---|
| active substance | 80 mg |
| lactose | 55 mg |
| corn starch | 190 mg |
| microcrystalline cellulose | 35 mg |
| polyvinylpyrrolidone | 15 mg |
| sodium-carboxymethyl starch | 23 mg |
| magnesium stearate | 2 mg |
| | |
| total | 400 mg |

The finely ground active substance, some of the corn starch, lactose, microcrystalline cellulose and polyvinylpyrrolidone are mixed together, the mixture is screened and worked with the remaining corn starch and water to form a granulate which is dried and screened. The sodiumcarboxymethyl starch and the magnesium stearate are added and mixed in and the mixture is compressed to form tablets of a suitable size.

C) Ampoule solution

| | |
|---|---|
| active substance | 50 mg |
| sodium chloride | 50 mg |
| water for inj. | 5 ml |

The active substance is dissolved in water at its own pH or optionally at pH 5.5 to 6.5 and sodium chloride is added to make it isotonic. The solution obtained is filtered free from pyrogens and the filtrate is transferred under aseptic conditions into ampoules which are then sterilised and sealed by fusion. The ampoules contain 5 mg, 25 mg and 50 mg of active substance.

D) Injectable solutions

Example 1

| | |
|---|---|
| Active substance | 2 mg/ml |
| Hydrochloric acid 1N | 6.8 µl |
| NaCl | 0.009 g/ml |
| WFI (water for injection) ad | 1 ml |
| pH | 4.5 |
| mOsmol / kg | 295 |

Example 2

| | |
|---|---|
| Active substance | 10.0000 g |
| Hydrochloric acid 1N | 36.6735 g |
| NaCl | 45.0000 g |
| WFI (water for injection) ad | 4934.8265 g |
| pH | 4.3 |
| mOsmol / kg | 300 |

Example 3

| | |
|---|---|
| Active substance | 500 mg |
| Hydrochloric acid 1N | 1.6 ml |
| NaCl | 450 mg |
| WFI (water for injection) ad | 50 ml |
| pH | 4.0 |
| mOsmol / kg | 290 |

Example 4

| | |
|---|---|
| Active substance | 0.5 g |
| Hydrochloric acid 1N | 1.705 µl |
| NaCl | 9 mg |
| WFI (water for injection) ad | 1 ml |
| pH | 4.8 |
| mOsmol / kg | 285 |

Example 5

| | |
|---|---|
| Active substance | 1 mg |
| Hydrochloric acid 1N | 3.6125 µl |
| NaCl | 0.009 g |
| WFI (water for injection) ad | 1 ml |
| pH | 4.8 |
| mOsmol / kg | 295 |

Example 6

| | |
|---|---|
| Active substance | 2 mg |
| Phosphoric acid (85%) | 0.440 µml |
| NaCl | 9 mg |
| WFI (water for injection) ad | 1 ml |
| pH | 4.0 |
| mOsmol / kg | 298 |

Example 7

| | |
|---|---|
| Active substance | 100 mg |
| Acetic acid | 16.4 µl |
| Dextrose | 2.5 g |
| WFI (water for injection) ad | 50 ml |
| pH | 4.4 |
| mOsmol / kg | 305 |

Example 8

| | |
|---|---|
| Active substance | 10 mg |
| Tartaric acid | 4.32 mmmg |
| Mannit | 0.25 g |
| WFI (water for injection) ad | 5 ml |
| pH | 4.0 |
| mOsmol / kg | 298 |

For the sake of completeness, a process for the manufacture of the compound (12B) is described hereinafter. This method is to be understood as an illustration of the invention.

Synthesis Example

Abbreviations:

| | |
|---|---|
| CH | cyclohexane |
| DCM | dichloromethane |
| EA | ethyl acetate |
| MeOH | methanol |
| $^1$H-NMR | proton nuclear magnet resonance |
| HPLC | high pressure liquid chromatography |
| MPLC | medium pressure liquid chromatography |
| TLC | thin layer chromatography |

Synthesis of Compound (12B)

Step 1

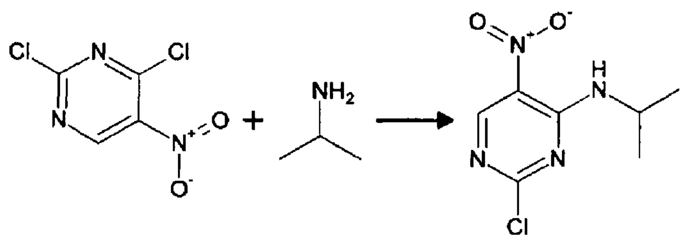

(1a) (2a) (3a)

Compound (1a), 2,4-dichloro-5-nitropyrimidine, (100 g, 0.52 mol) is dissolved in 1.0 L cyclohexane and potassium carbonate (83 g, 0.60 mol) is added. The resulting suspension is stirred at a temperature between 5 and 15°C and compound (2a), isopropylamine, (44.2 ml, 0.52 mol) is slowly added. After complete addition stirring is continued under warm up of the reaction mixture to room temperature. Water (400 mL) is added (caution: exothermic reaction). The reaction mixture is filtered. Ethyl acetate (400 mL) is added to the filtrate. The organic phase is separated, dried and evaporated.
Yield: 90.9 g (81 % of theory) of compound (3a) as a brown crystalline solid.
$^1$H-NMR confirmed the structure of compound (3a).

Step 2

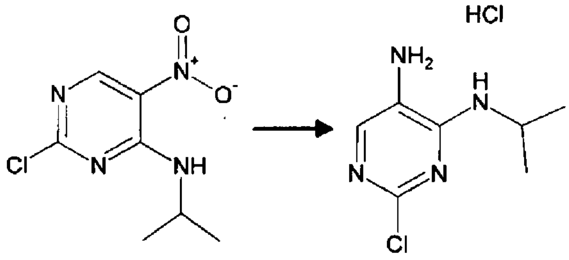

(3a) (4a)

Compound (3a) (90.9 g, 0.420 mol), tetrahydrofuran (840 mL, abs.), Pt/C 5% (9.0 g, 42 mmol) and vanadyl acetylacetonate (4.5 g, 16 mmol) are added in a Parr apparatus and shaken under a hydrogen pressure of 50 psi (1 psi corresponds to 6894.8 pascal) at 20 °C to 40 °C for several hours until the reduction of the nitro group is complete (TLC control: silica gel, CH : EE = 1 : 1). The catalyst is removed and the solvent is evaporated under reduced pressure. The crude product is dissolved in a mixture of tetrahydrofuran (100 mL) and isopropanol (120 mL) and transferred into a three necked flask. Trimethylchlorosilane (54 ml) is added dropwise and the hydrochlorid precipitates. The suspension is stirred for 16 hours. The precipitate is suction filtered and dried. Yield: 54.8 g (59 % of theory) of compound (4a) as brown crystalline solid.

Step 3

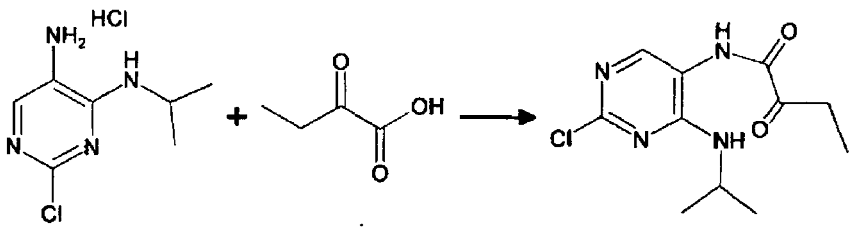

(4a) (5a) (6a)

1-ethyl-3-(3-dimethylaminopropyl) carbodiimide hydrochloride (8.7 g, 45 mmol) is added to a solution of Compound (4a) (10.0 g, 45 mmol) and compound (5a), 2-ketobutyric acid, (4.7 g, 46 mmol) in n-methyl-2-pyrrolidone (80 mL) under inert gas atmosphere and ice bath cooling. After complete dissolution the ice bath is removed and stirring is continued for 16 hours. Additional 2-ketobutyric acid (462 mg, 4 mmol) and 1-ethyl-3-(3-dimethylaminopropyl) carbodiimide hydrochloride (860 mg, 4 mmol) are added and the reaction mixture is stirred for about 2 to 3 hours at room temperature. The reaction mixture is poured into water (50 mL) under cooling. Ethyl acetate (150 mL) is added. The organic phase is separated, washed with water (50 mL) and saturated sodium cloride solution (2 x, 25 mL), dried over sodium sulfate and filtered. The solvent is evaporated under reduced pressure. The crude product is purified via MPLC (silica gel, CH : EE = 2 : 1).
Yield: 6.65g (55% of theory) of compound (6a) as pink crystalline solid.

Step 4

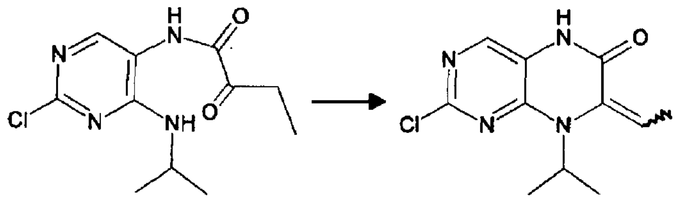

(6a) (7a)

Compound (6a) (6.65g, 24.6 mmol) in toluene (150 ml) is heated under reflux in a water separator apparatus for 16 hours. TLC (silica gel, DCM : MeOH = 9 : 1 + $NH_3$) confirmed that the starting material disappeared. The solvent is evaporated under reduced pressure. Yield: 5.6 g (90 % of theory) of compound (7a) as beige crystalline solid.
$^1$H-NMR confirmed the presence of a mixture of isomers.

Step 5

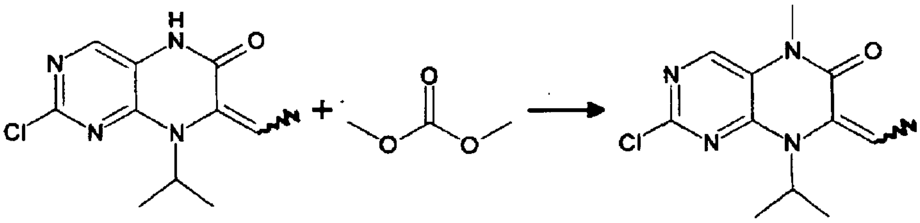

(7a) (8a) (9a)

Compound (7a) (5.6 g, 22 mmol), potassium carbonate powder (4.6 g, 33 mmol) and compound (8a), namely dimethylcarbonate (22.4 ml, 0.266 mol) are heated in an autoclave to 130 °C for several hours. The completion of the reaction is confirmed by TLC (silica gel, CH : EE = 1 : 2). Water (demineralized, 50 mL) and ethyl acetate (50 mL) are added. The organic phase is separated and washed (2x) with sodium chloride solution (25 mL), The organic phase is dried over sodium sulfate and filtered. The solvent is evaporated under reduced pressure. The crude product is purified via MPLC (silica gel, DCM : MeOH = 100 : 1).
Yield: 5.14 g (87 % of theory) of compound (9a) as brown oil.
$^1$H-NMR confirmed the presence of a mixture of isomers.

Step 6

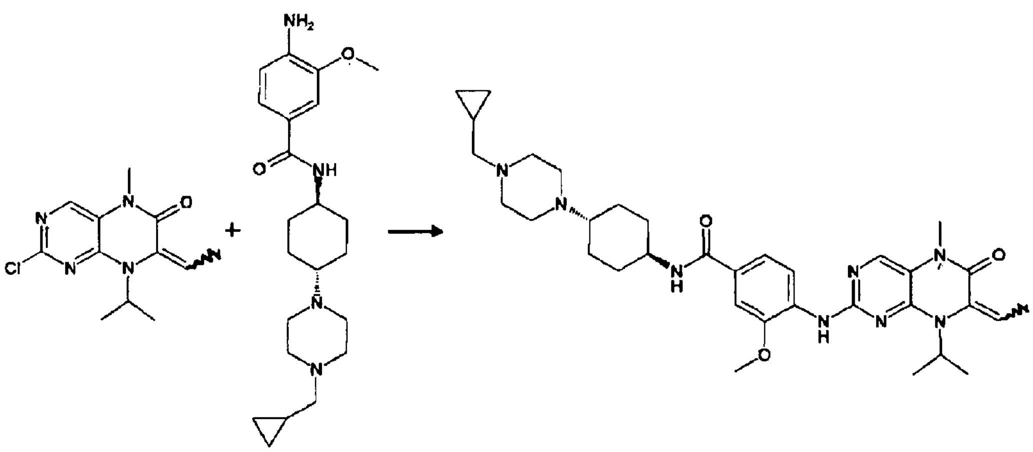

(9a) (10a) (11a)

Compound (9a) (4.7 g, 17.6 mmol), compound (10a) (6.45 g, 16.7 mmol), and p-toluene sulphonic acid hydrate (8.05 g, 41.7 mmol) in 4-methyl-2-pentanol (100 mL) are heated under reflux for 6 hours. The reaction mixture is allowed to cool to room temperature. Ethyl acetate and water are added. The ethyl acetate phase is separated and reextracted with a small amount of water. Dichloromethane is added to the combined aqueous phases. NaOH (1 N) is added to make the aqueous phase alkaline. The dichloromethane phase is separated and the alkaline aqueous phase is reextracted once with dichloromethane. The combined dichloromethane phases are dried over sodium sulfate and filtered. The solvent is evaporated under reduced pressure.
The crude product is purified via MPLC (silica gel, DCM : MeOH : $NH_3$ = 9 : 1 : 0.1). Yield: 5.04 g (49 % of theory) of compound (11a) as yellow solid.
$^1$H-NMR confirmed the presence of a mixture of isomers.

<u>Step 7</u>

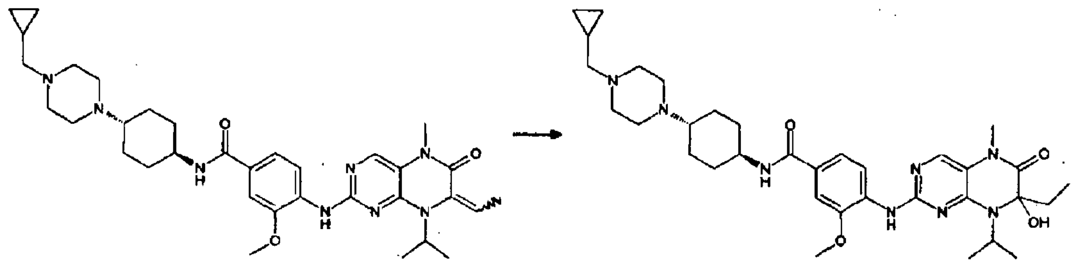

(11a) (12a)

A solution of compound (11a) (4.75 g, 7.70 mmol) in 0.5 molar sulphuric acid (62 mL, 31.0 mmol) is heated under reflux for several hours. The progress of the reaction is controlled via TLC (silica gel, DCM : MeOH = 4 : 1 + $NH_3$) The reaction mixture is diluted with water (demineralised, 100 mL) and added dropwise to a solution of 1 molar NaOH (62 ml, 62 mmol) in water (demineralised, 100 mL) under cooling in an ice bath. The precipitate is filtered washed with water and subsequently with petroleum ether. The wet crude product is dissolved in DCM and the phases are separated. The organic phase is evaporated under reduced pressure. The crude product is purified via MPLC (silica gel, DCM : MeOH : $NH_3$ = 9 : 1 : 0.1).
Yield: 2.6 g (53 % of theory) of compound (12a) as yellowish solid.
The product can be further purified via preparative HPLC.

## Claims

1. Compounds of general formula (12)

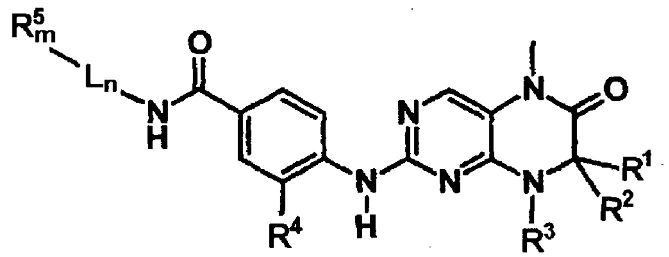

wherein

$R^1$ denotes hydrogen or $C_1$-$C_6$-alkyl, optionally substituted with one or more halogens,
$R^2$ denotes an -OH group, or

$R^3$ denotes a group selected from among $C_1$-$C_5$-alkyl and $C_3$-$C_7$-cycloalkyl,
$R^4$ denotes a group selected from among hydrogen, OMe, OH, Me, Et, Pr, OEt, NHMe, $NH_2$, F, CL, Br, O-propargyl, O-butynyl, CN, SMe, $NMe_2$, $CONH_2$, ethynyl, propynyl, butynyl and allyl,
L denotes a linker selected from among phenyl, phenylmethyl, cyclohexyl and optionally branched $C_1$-$C_6$-alkyl,
n denotes 0 or 1
m denotes 1 or 2
$R^5$ denotes a group selected from piperidinyl, pyrrolidinyl and piperazinyl the heteroatoms of the groups of $R^5$ may be optionally substituted with $R^8$ and/or $R^9$,
$R^8$, $R^9$ denote unsubstituted substituents at the heteroatoms of the groups of $R^5$, which may be identical or different, and denote either hydrogen or a group selected from among $C_1$-$C_6$-alkyl, $C_1$-$C_4$-alkyl-$C_3$-$C_{10}$-cycloalkyl and $C_3$-$C_{10}$-cycloalkyl,
optionally in the form of the tautomers, the racemates, the enantiomers, the diastereomers and the mixtures thereof, and optionally the salts thereof.

**2.** Compounds according to claim 1, wherein

$R^2$ and $R^3$ are as hereinbefore defined, and
$R^1$ denotes an ethyl group,
$R^4$ denotes a group selected from among hydrogen, OH, OMe and OEt,
L denotes a linker selected from $C_3$-$C_6$-cycloalkyl,
n denotes 0 or 1
m denotes 1
$R^5$ denotes a group which is bound to L via a nitrogen atom, selected from among piperidinyl and piperazinyl, the heteroatoms of the groups of $R^5$ may be optionally substituted with $R^8$ and/or $R^9$, and
$R^8$, $R^9$ denote unsubstituted substituents at the heteroatoms of the groups of $R^5$, selected from among $C_1$-$C_6$-alkyl, $C_1$-$C_4$-alkyl-$C_3$-$C_{10}$-cycloalkyl, and $C_3$-$C_{10}$-cycloalkyl,
optionally in the form of the tautomers, the racemates, the enantiomers, the diastereomers and the mixtures thereof, and optionally the salts thereof.

**3.** Compound of formula (12) according to one of claims 1 to 2, optionally in form of its tautomers, racemates, enantiomers, diastereomers and the mixtures thereof and optionally in form of the salts thereof, for use as a pharmaceutical composition, preferably a pharmaceutical composition with an antiproliferative activity.

**4.** Use of a compound of formula (12) according to one of claims 1 to 2, optionally in form of its tautomers, racemates, enantiomers, diastereomers and the mixtures thereof and optionally in form of the salts thereof, for preparing a pharmaceutical composition for the treatment and/or prevention of cancer, infections, inflammatory and autoimmune diseases.

**5.** Use of a compound of formula (12) according to one of claims 1 to 2, optionally in form of its tautomers, racemates, enantiomers, diastereomers and the mixtures thereof and optionally in form of the salts thereof, for preparing a pharmaceutical composition for the treatment of diseases **characterized by** an abnormal cell proliferation in a human or non-human mammalian body by inhibition of polo-like kinases as mitotic regulators, preferably in case the polo-like kinase is PLK-1.

**6.** Use of a compound according to any one of claims 1 to 2, optionally in form of its tautomers, racemates, enantiomers, diastereomers and the mixtures thereof and optionally in form of the salts thereof, for the preparation of a pharmaceutical composition for the treatment of

- autoimmune disorders, particularly selected from the group consisting of Amyloidosis, Systemic lupus erythematosus, Rheumatoid Arthritis, Crohn's disease, multiple sclerosis, systemic sclerosis (scleroderma), mixed connective tissue disease, Sjögren's syndrome, ankylosing spondylitis, autoimmune vasculitis, Behcet's syndrome, psoriasis, autoimmune arthritis, sarcoidosis and diabetes mellitus; or
- fungous diseases, particularly selected from the group consisting of candidiasis, cryptococcosis, aspergillosis, mucormycosis, tinea, dermatophytosis, histoplasmosis, blastomycosis, coccidiosis and pneumocystis; or
- cancer selected from the group consisting of carcinomas, sarcomas, melanomas, myeloma, hematologic neoplasias such as leukaemia, lymphomas, and childhood cancers.

**7.** Pharmaceutical preparations, containing as active substance one or more compounds of general formula (12)

according to one of claims 1 to 2 or the pharmacologically acceptable salts thereof, optionally combined with conventional excipients and/or carriers.

**8.** Compound according to claim 1 of formula (12B),

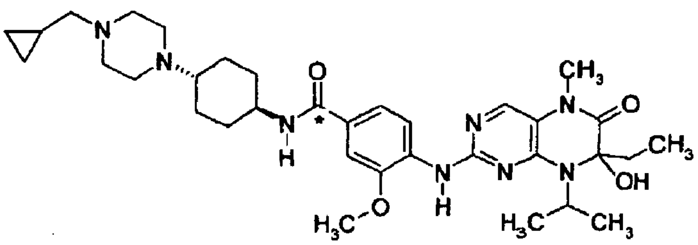

optionally in the form of the racemates, the enantiomers, the diastereomers and the mixtures thereof, and optionally the salts thereof.

## Patentansprüche

**1.** Verbindungen der allgemeinen Formel (12)

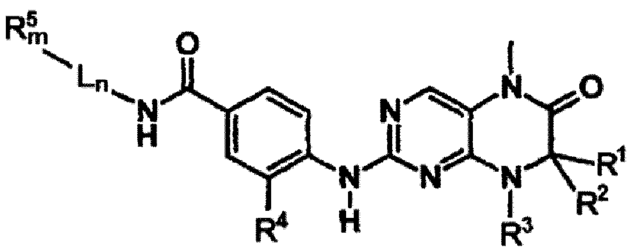

wobei

$R^1$ Wasserstoff oder $C_1$-$C_6$-Alkyl, gegebenenfalls mit ein oder mehreren Halogenen substituiert, bezeichnet,
$R^2$ bezeichnet eine -OH-Gruppe, oder
$R^3$ bezeichnet eine Gruppe, ausgewählt aus $C_1$-$C_5$-Alkyl und $C_3$-$C_7$-Cycloalkyl,
$R^4$ bezeichnet eine Gruppe, ausgewählt aus Wasserstoff, OMe, OH, Me, Et, Pr, OEt, NHMe, NH, F, CL, Br, O-Propargyl, O-Butinyl, CN, SMe, $NMe_2$, $CONH_2$, Ethinyl, Propinyl, Butinyl und Allyl,
L bezeichnet einen Linker bzw. eine Verbindungsgruppe, ausgewählt aus Phenyl, Phenylmethyl, Cyclohexyl und gegebenenfalls verzweigtem $C_1$-$C_6$-Alkyl,
n bezeichnet 0 oder 1
m bezeichnet 1 oder 2
$R^5$ bezeichnet eine Gruppe, ausgewählt aus Piperidinyl, Pyrrolidinyl und Piperazinyl, wobei die Heteroatome der Gruppen $R^5$ gegebenenfalls mit $R^8$ und/oder $R^9$ substituiert sein können,
$R^8$, $R^9$ bezeichnen unsubstituierte Substituenten an den Heteroatomen der Gruppen $R^5$, die gleich oder verschieden sein können, und bezeichnen entweder Wasserstoff oder eine Gruppe, ausgewählt aus $C_1$-$C_6$-Alkyl, $C_1$-$C_4$-Alkyl-$C_3$-$C_{10}$-cycloalkyl und $C_3$-$C_{10}$-Cycloalkyl,
gegebenenfalls in Form der Tautomeren, der Racemate, der Enantiomeren, der Diastereomeren und der Mischungen hiervon und gegebenenfalls der Salze hiervon.

**2.** Verbindungen nach Anspruch 1, wobei $R^2$ und $R^3$ wie hier zuvor definiert sind, und

$R^1$ bezeichnet eine Ethyl-Gruppe,
$R^4$ bezeichnet eine Gruppe, ausgewählt aus Wasserstoff, OH, OMe und OEt,
L bezeichnet einen Linker bzw. eine Verbindungsgruppe, ausgewählt aus $C_3$-$C_6$-Cycloalkyl,
n bezeichnet 0 oder 1

m bezeichnet 1
$R^5$ bezeichnet eine Gruppe, die über ein Stickstoffatom an L gebunden ist, ausgewählt aus Piperidinyl und Piperazinyl, wobei die Heteroatome der Gruppen $R^5$ gegebenenfalls mit $R^8$ und/oder $R^9$ substituiert sein können, und
$R^8$, $R^9$ bezeichnen unsubstituierte Substituenten an den Heteroatomen der Gruppen $R^5$, ausgewählt aus $C_1$-$C_6$-Alkyl, $C_1$-$C_4$-Alkyl-$C_3$-$C_{10}$-cycloalkyl und $C_3$-$C_{10}$-Cycloalkyl,
gegebenenfalls in Form der Tautomeren, der Racemate, der Enantiomeren, der Diastereomeren und der Mischungen hiervon und gegebenenfalls der Salze hiervon.

3. Verbindung der Formel (12) nach einem der Ansprüche 1 bis 2, gegebenenfalls in Form ihrer Tautomeren, Racemate, Enantiomeren, Diastereomeren und den Mischungen hiervon und gegebenenfalls in Form der Salze hiervon, zur Verwendung als eine pharmazeutische Zusammensetzung, bevorzugt eine pharmazeutische Zusammensetzung mit einer antiproliferativen Wirksamkeit.

4. Verwendung einer Verbindung der Formel (12) nach einem der Ansprüche 1 bis 2, gegebenenfalls in Form ihrer Tautomeren, Racemate, Enantiomeren, Diastereomeren und den Mischungen hiervon und gegebenenfalls in Form der Salze hiervon, zur Herstellung einer pharmazeutischen Zusammensetzung zur Behandung und/oder Vorbeugung von Krebs, Infektionen, inflammatorischen und Autoimmun-Erkrankungen.

5. Verwendung einer Verbindung der Formel (12) nach einem der Ansprüche 1 bis 2, gegebenenfalls in Form ihrer Tautomeren, Racemate, Enantiomeren, Diastereomeren und den Mischungen hiervon und gegebenenfalls in Form der Salze hiervon, zur Herstellung einer pharmazeutischen Zusammensetzung zur Behandung von Erkrankungen, **gekennzeichnet durch** eine abnormale Zellproliferation in einem menschlichen oder nicht-menschlichen Säugetierkörper **durch** Inhibierung der poloartigen Kinasen als mitotische Regulatoren, bevorzugt in dem Falle, dass die poloartige Kinase PLK-1 darstellt.

6. Verwendung einer Verbindung nach irgendeinem der Ansprüche 1 bis 2, gegebenenfalls in Form ihrer Tautomeren, Racemate, Enantiomeren, Diastereomeren und den Mischungen hiervon und gegebenenfalls in Form der Salze hiervon, zur Herstellung einer pharmazeutischen Zusammensetzung zur Behandung von

- Autoimmunstörungen, insbesondere ausgewählt aus der Gruppe, bestehend aus Amyloidose, systemischem Lupus erythematodes, rheumatoider Arthritis, Crohnscher Erkrankung, Multipler Sklerose, systemischer Sklerose (Scleroderma), gemischter Bindegewebeerkrankung, Sjogren-Syndrom, Spondylitis ankylosum, autoimmuner Vasculitis, Behcet-Syndrom, Psoriasis, autoimmuner Arthritis, Sarkoidose und Diabetes mellitus; oder
- Pilzerkrankungen, insbesondere ausgewählt aus der Gruppe, bestehend aus Candidiase, Cryptococcose, Aspergillose, Mukormycose, Tinea, Dermatophytose, Histoplasmose, Blastomycose, Coccidiose und Pneumocyste; oder
- Krebs, ausgewählt aus der Gruppe, bestehend aus Karzinomen, Sarkomen, Melanomen, Myelom, hämatologischer Neoplasie, wie Leukämie, Lymphomen und Krebs bei Kindern.

7. Pharmazeutische Zubereitungen, enthaltend als aktive Substanz bzw. Wirksubstanz ein oder mehrere Verbindungen der allgemeinen Formel (12) nach einem der Ansprüche 1 bis 2 oder die pharmakologisch akzeptablen bzw. annehmbaren Salze hiervon, gegebenenfalls kombiniert mit herkömmlichen Hilfsstoffen und/oder Trägern.

8. Verbindung nach Anspruch 1 der Formel (12B),

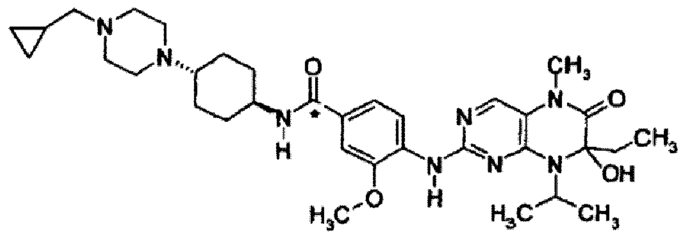

gegebenenfalls in Form der Racemate, der Enantiomeren, der Diastereomeren und der Mischungen hiervon und gegebenenfalls der Salze hiervon.

## Revendications

**1.** Composés de formule générale (12)

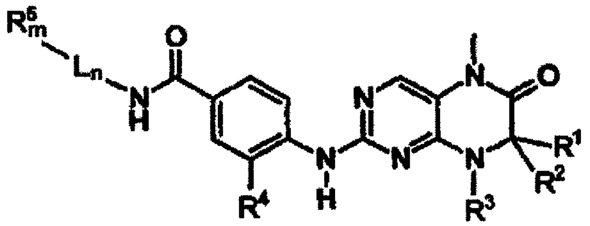

dans laquelle

$R^1$ désigne un atome d'hydrogène ou un groupe $C_1$-$C_6$-alkyle, éventuellement substitué par un ou plusieurs atomes d'halogène,
$R^2$ désigne un groupe -OH, ou
$R^3$ désigne un groupe choisi parmi les groupes $C_1$-$C_5$-alkyle et $C_3$-$C_7$-cycloalkyle,
$R^4$ désigne un groupe choisi parmi un atome d'hydrogène, les groupes OMe, OH, Me, Et, Pr, OEt, NHMe, $NH_2$, F, CL, Br, O-propargyle, O-butynyle, CN, SMe, $NMe_2$, $CONH_2$, éthynyle, propynyle, butynyle et allyle,
L désigne un lieur choisi parmi les groupes phényle, phénylméthyle, cyclohexyle et $C_1$-$C_6$-alkyle éventuellement ramifié,
n désigne 0 ou 1
m désigne 1 ou 2
$R^5$ désigne un groupe choisi parmi les groupes pipéridinyle, pyrrolidinyle et pipérazinyle, les hétéroatomes des groupes de $R^5$ peuvent être éventuellement substitués par $R^8$ et/ou $R^9$,
$R^8$, $R^9$ désignent des substituants non substitués au niveau des hétéroatomes des groupes de $R^5$, qui peuvent être identiques ou différents, et indiquent soit un atome d'hydrogène soit un groupe choisi parmi les groupes $C_1$-$C_6$-alkyle, $C_1$-$C_4$-alkyl-$C_3$-$C_{10}$-Cycloalkyle et $C_3$-$C_{10}$-cycloalkyle,
éventuellement sous la forme de leurs tautomères, leurs racémates, leurs énantiomères, leurs diastéréomères et leurs mélanges, et éventuellement leurs sels.

**2.** Composés selon la revendication 1, dans lesquels

$R^2$ et $R^3$ sont tels que définis ci-avant, et
$R^1$ désigne un groupe éthyle,
$R^4$ désigne un groupe choisi parmi un atome d'hydrogène, les groupes OH, OMe et OEt,
L désigne un lieur choisi parmi un groupe $C_3$-$C_6$-cycloalkyle,
n désigne 0 ou 1
m désigne 1
$R^5$ désigne un groupe qui est lié à L par l'intermédiaire d'un atome d'azote, choisi parmi les groupes pipéridinyle et pipérazinyle, les hétéroatomes des groupes de $R^5$ peuvent être éventuellement substitués par $R^8$ et/ou $R^9$, et
$R^8$, $R^9$ désignent des substituents non substitués au niveau des hétéroatomes des groupes de $R^5$, choisis parmi les groupes $C_1$-$C_6$-alkyle, $C_1$-$C_4$-alkyl-$C_3$-$C_{10}$-cycloalkyle et $C_3$-$C_{10}$-cycloalkyle,
éventuellement sous la forme de leurs tautomères, leurs racémates, leurs énantiomères, leurs diastéréomères et leurs mélanges, et éventuellement leurs sels.

**3.** Composé de formule (12) selon l'une quelconque des revendications 1 à 2, éventuellement sous la forme de ses tautomères, racémates, énantiomères, diastéréomères et leurs mélanges et éventuellement sous la forme de ses sels, pour une utilisation en tant que composition pharmaceutique, de préférence une composition pharmaceutique avec une activité antiproliférative.

**4.** Utilisation d'un composé de formule (12) selon l'une quelconque des revendications 1 à 2, éventuellement sous la forme de ses tautomères, racémates, énantiomères, diastéréomères et leurs mélanges et éventuellement sous la forme de ses sels, pour la préparation d'une composition pharmaceutique destinée au traitement et/ou à la prévention d'un cancer, d'infections, de maladies inflammatoires et auto-immunes.

5. Utilisation d'un composé de formule (12) selon l'une quelconque des revendications 1 à 2, éventuellement sous la forme de ses tautomères, racémates, énantiomères, diastéréomères et leurs mélanges et éventuellement sous la forme de ses sels, pour la préparation d'une composition pharmaceutique destinée au traitement de maladies **caractérisées par** une prolifération cellulaire anormale dans un corps humain ou de mammifère non humain par inhibition des kinases de type polo en tant que régulateurs mitotiques, de préférence dans le cas où la kinase de type polo est la PLK-1.

6. Utilisation d'un composé de formule (12) selon l'une quelconque des revendications 1 à 2, éventuellement sous la forme de ses tautomères, racémates, énantiomères, diastéréomères et leurs mélanges et éventuellement sous la forme de ses sels, pour la préparation d'une composition pharmaceutique destinée au traitement

   - de troubles auto-immuns, choisis particulièrement dans le groupe consistant en l'amyloïdose, le lupus érythémateux systémique, la polyarthrite rhumatoïde, la maladie de Crohn, la sclérose en plaques, la sclérose systémique (sclérodermie), la connectivité mixte, le syndrome de Sjögren, la spondylarthrite ankylosante, la vascularite auto-immune, le syndrome de Behçet, le psoriasis, l'arthrite auto-immune, la sarcoïdose et le diabète sucré ; ou
   - de maladies fongiques, choisies particulièrement dans le groupe consistant en la candidose, la cryptococcose, l'aspergillose, la mucormycose, la teigne, la dermatophytose, l'histoplasmose, la blastomycose, la coccidiose et la pneumocystose ; ou
   - d'un cancer choisi dans le groupe consistant en les carcinomes, les sarcomes, les mélanomes, le myélome, les néoplasies hématologiques telles que la leucémie, les lymphomes, et les cancers de l'enfance.

7. Préparations pharmaceutiques, contenant en tant que substance active un ou plusieurs composés de formule générale (12) selon l'une quelconque des revendications 1 à 2 ou leurs sels pharmacologiquement acceptables, éventuellement combinés avec des excipients et/ou des supports traditionnels.

8. Composé selon la revendication 1 de formule (12B)

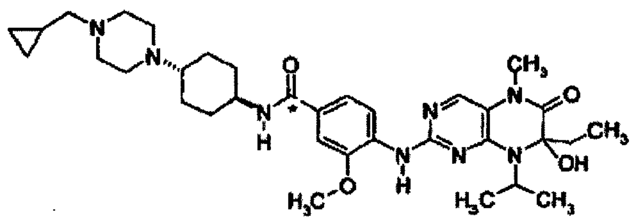

éventuellement sous la forme de ses racémates, ses énantiomères, ses diastéréomères et leurs mélanges, et éventuellement ses sels.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004014899 A
- WO 01019825 A1
- WO 06021547 A
- WO 06021378 A
- WO 06021379 A
- WO 06005510 A
- WO 09112524 A
- WO 2004076454 A1
- WO 03020722 A1
- WO 2006018185 A2
- WO 2006018182 A
- WO 0043369 A
- WO 0043372 A


### Non-patent literature cited in the description


- **GLOVER et al.** *Genes Dev.,* 1998, vol. 12, 3777-87
- **QIAN et al.** *Mol Biol Cell,* 2001, vol. 12, 1791-9
- **WOLF et al.** *Oncogene,* 1997, vol. 14, 543-549
- **KNECHT et al.** *Cancer Res.,* 1999, vol. 59, 2794-2797
- **WOLF et al.** *Pathol Res Pract.,* 2000, vol. 196, 753-759
- **WEICHERT et al.** *Br. J. Cancer,* 2004, vol. 90, 815-821
- **ITO et al.** *Br. J. Cancer,* 2004, vol. 90, 414-418
- **TAKAHASHI et al.** *Cancer Sci.,* 2003, vol. 94, 148-152
- **GLOVER et al.** *Genes & Dev.,* 1998, vol. 12, 3777-3787
- **NIGG et al.** *Curr. Opin. Cell Biol.,* 1998, vol. 10, 776-783
- **DONALDSON et al.** *J Cell Sci.,* 2001, vol. 114, 2357-2358